(19) Europäisches Patentamt
European Patent Office
Office européen des brevets

(11) **EP 1 414 945 B1**

(12) **EUROPEAN PATENT SPECIFICATION**

(45) Date of publication and mention
of the grant of the patent:
**21.01.2009 Bulletin 2009/04**

(51) Int Cl.:
**C12N 5/06** (2006.01)     **C12N 5/10** (2006.01)
**G01N 33/50** (2006.01)

(21) Application number: **02755137.3**

(22) Date of filing: **09.08.2002**

(86) International application number:
**PCT/GB2002/003675**

(87) International publication number:
**WO 2003/014334 (20.02.2003 Gazette 2003/08)**

(54) **CELL CULTURE METHOD FOR OBTAINING PROSTATE-LIKE ACINI**

ZELLKULTURMETHODE ZUR ERZEUGUNG VON PROSTATA-ÄHNLICHEN ACINI

PROCEDE DE CULTURE CELLULAIRE PERMETTANT D'OBTENIR DES ACINI DE TYPE PROSTATIQUE

(84) Designated Contracting States:
**AT BE BG CH CY CZ DE DK EE ES FI FR GB GR IE IT LI LU MC NL PT SE SK TR**

(30) Priority: **09.08.2001 US 311202 P**
**10.08.2001 GB 0119533**

(43) Date of publication of application:
**06.05.2004 Bulletin 2004/19**

(73) Proprietor: **Procure Therapeutics Limited York YO10 5DG (GB)**

(72) Inventors:
• **LANG, Shona,**
**The University of York**
**York YO10 5YW (GB)**
• **MAITLAND, Norman,**
**The University of York**
**York YO10 5YW (GB)**

(74) Representative: **Harrison, Michael Robert et al Gilholm Harrison Limited**
**Marlborough House**
**Westminster Place**
**York Business Park**
**Nether Poppleton**
**York**
**YO26 6RW (GB)**

(56) References cited:
• **LANG S H ET AL: "IN VITRO MODELLING OF EPITHELIAL AND STROMAL INTERACTIONS IN NON-MALIGNANT AND MALIGNANT PROSTATES" BRITISH JOURNAL OF CANCER, LONDON, GB, vol. 82, no. 4, 2000, pages 990-997, XP000915839 ISSN: 0007-0920**
• **HASHIBA TAKAHUMI ET AL: "Serum-free coculture of stromal and epithelial cells from benign prostatic hyperplasia with keratinocyte growth factor." UROLOGIA INTERNATIONALIS, vol. 64, no. 4, June 2000 (2000-06), pages 209-212, XP001119416 ISSN: 0042-1138**
• **COLLINS A T ET AL: "Androgen and oestrogen responsiveness of stromal cells derived from the human hyperplastic prostate: Oestrogen regulation of the androgen receptor." JOURNAL OF ENDOCRINOLOGY, vol. 143, no. 2, 1994, pages 269-277, XP001117921 ISSN: 0022-0795**
• **MA YU-LIN ET AL: "Reconstruction of prostatic acinus-like structure from ventral and dorsolateral prostatic epithelial cells of the rat in three-dimensional collagen gel matrix culture." JOURNAL OF UROLOGY, vol. 157, no. 3, 1997, pages 1025-1031, XP001117923 ISSN: 0022-5347 cited in the application**
• **HALL JUNE A ET AL: "Primary prostate stromal cells modulate the morphology and migration of primary prostate epithelial cells in type 1 collagen gels." CANCER RESEARCH, vol. 62, no. 1, 1 January 2002 (2002-01-01), pages 58-62, XP002220106 January 1, 2002 ISSN: 0008-5472**

**(Cont. next page)**

- LANG SHONA H ET AL: "Experimental prostate epithelial morphogenesis in response to stroma and three-dimensional Matrigel culture." CELL GROWTH & DIFFERENTIATION, vol. 12, no. 12, December 2001 (2001-12), pages 631-640, XP001117481 ISSN: 1044-9523

- PRICE ET AL: 'Immortalization of a human prostate stromal cell line using a recombinant retroviral approach' J. UROLOGY vol. 164, December 2000, pages 2145 - 2150, XP005554240

**Description**

The invention relates to a method for the formation of prostate acini; cancerous acini derived by said method; cancerous cells or cell-lines derived from said acini; methods to identify agents capable of inhibiting the proliferation and/or motility of cancerous prostate cells; and methods to identify novel markers of cancerous prostate cell differentiation.

Prostate cancer is a leading cause of cancer related deaths in men. The prostate is a male sex gland located in the lower pelvic just below the bladder, the prostate surrounds the urethra, producing the fluid component of semen and helping to control the flow of urine. Enlargement of the prostate is common with age and is a non-maligant condition. Symptoms include, blood in the semen or the urine, frequent pain or stiffness in the lower back, hips or upper thigh. Prostate cancer is a disease of uncontrolled cell proliferation which results in the formation of tumours. The tumours may be primary (ie located in the organ of origin) or secondary (ie tumours which form in other organs due to the ability of cancerous cells to move and invade other tissues via the circulatory system).

Prostate cancer can be relatively harmless or extremely aggressive. Some prostate tumours are slow growing and cause few clinical symptoms. Aggressive prostate tumours spread rapidly to the lymph nodes and other organs, especially bone. It is known that the growth of prostate cancer can be inhibited by blocking the supply of male hormones such as testosterone. However, prostate cancers eventually develop and become independent of male sex hormones (ie they become androgen-independent prostate cancer cells). These cells are linked with aggressive, malignant prostate cancer.

All male mammals have a prostate gland but only humans and dogs are known to naturally develop prostate cancer.

There are a number of model systems which purport to be useful models for the study of prostate cell biology. US 5, 874, 305 describes a prostate cell-line which grows in monolayer. The cell-line is androgen- independent (not sensitive to the addition of male sex hormones) prostate cancer cell-line. These cells are know in the art and correlate with aggressive tumour forming ability. The cell-line is grown in monolayer and therefore is not an authentic representation of the development of prostate tumours. It would be desirable to target prostate cancer cells before they become androgen independent and more recalcitrant to therapy.

In men over the age of 40 the prostate represents a major medical problem since both benign prostatic hyperplasia and prostatic carcinoma are becoming increasingly prevalent (Boyle. 1994)). Both the epithelia and stroma play roles in the advancement of these diseases therefore good models to study the interaction of these cell types is very important. For example, US 5, 917, 124 and US 5, 907, 078 disclose transgenic murine models of prostate cancer. Each patent discloses the use of prostate specific promoters to drive expression of SV40 T antigen in transgenic mice. The prostate cells become transformed to reflect the aggressive form of prostate cancer. These transgenic animals do not necessarily reflect the development of a prostate tumour since not all tumours are virally induced. Also, as with studying androgen-repressed prostate tumour cells, the transgenic models only reflect the aggressive prostate tumours of late stage prostate cancer.

Although animal models exist to study prostatic growth and differentiation (Timms, Lee, Aumüller, & Seitz. 1995)) there are no human three dimensional models which successfully employ stromal and epithelial cultures to produce morphological and functional differentiation of the prostate. Such models are essential to understand the normal physiology of the prostate and better understand disease development.

In the prostate, rat epithelial cultures have been grown within both collagen gels (Ma, Fujiyama, Masaki, & Sugihara. 1997) and Matrigel[tm] (Freeman, Bagli, Lamb, et al. 1994). In collagen, these formed acinus-like structures which secreted Prostate Specific Antigen whilst in Matrigel, spheroids of cell masses with no normal morphological and functional differentiation were produced. Normal human prostatic epithelial cell lines cultured in Matrigel showed acinus-like structures with lumen and PSA secretion (Webber, Bello, Kleinman, & Hoffman. 1997), however, primary cultures formed solid cell masses with little functional differentiation (Hudson, O'Hare, Watt, & Masters. 2000).

We have developed an *in vitro* cell culture method which provides a culture regime which allows prostate epithelial cells to form prostate-like-acini which closely resemble prostate acini found *in vivo.* Our method relies on a combination of serum, hormones and a suitable cell, matrix support which allows the epithelial cells to attach, proliferate, differentiate and form prostatic-like-acini. The system is able to support growth of cloned normal prostate epithelial cells as well as clones cancerous prostate cells, primary prostate epithelial cells, extends life span of cells such as P4, E6 and primary cancerous prostate cells to provide a 3D structure which reflects the *in vivo* state. The system is invaluable for the study of prostate cell differentiation and prostate cell transformation. It will provide a tool for use in the identification of agents effective at inhibiting the proliferation and metastasis of prostate cancer cells and also to identity novel markers, of prostate cell differentiation and transformation.

The invention relates to a cell culture method which combines a cell support matrix to which prostate cells attach,and proliferate and culture conditions which combine serum, stromal cell extract and hormones the combination of which promotes the formation of prostate like - acini with similar characteristics to *in vivo* acini.

According to a first aspect of the invention, there is provided an *in vitro* method for the formation of prostate-like acini comprising:

i) providing a cell culture vessel comprising:

a) prostate derived epithelial cells;
b) a collagen based support matrix to which the cells in (a) can attach and proliferate;
c) cell culture medium supplemented serum, a prostate stromal fraction comprising stomal cells and the hormones oestrogen and dihydrotestosterone, provided at 10ng/ml and $10^{-7}$ m respectively;

ii) providing conditions which promote the growth and differentiation of said prostate derived cells in said vessel.

"Vessel" is defined as any means suitable to contain the above described cell culture. Typically, examples of such a vessel is a petri dish; cell culture bottle or flask; multiwell culture dishes.

In a further preferred method of the invention the supplemented cell culture medium comprises a mixture of serum, stromal fraction, hormones and cell culture medium to which the prostate derived cells are added. Alternatively, or preferably, the stromal fraction is provided in a separate vessel, but in liquid contact with the other components of the supplemented cell culture medium. Typically the separate vessel is an insert or similar means which allows the cells contained in the stomal fraction to proliferate but prevents cell contact with the prostate derived cells contained in the vessel.

We have observed that a combination of components are required to maximise acini formation. In the presence of stroma, the number of spheroids formed approximately doubled and further increased with the addition of oestrogen and dihydrotestosterone. In addition, presentation of the stroma in the co-culture was examined by comparing stroma within an insert to that directly mixed with epithelia in a cell culture support (eg Matrigel), or added to the top of a preset gel. Our results indicated that stroma co-cultured within an insert produced maximal spheroid formation.

In a preferred method of the invention said prostate cells are epithelial cells, preferably human epithelial cells. Preferably said epthelial cells are derived from prostate glands which have been maintained as explants for at least 7 days.

We have observed that the use of epithelial cells from 7 day explants rather than freshly isolated led to greater acini forming efficiency and that the acini subsequently produced maintained in culture for longer periods.

In a preferred method of the invention, the prostate derived cells are normal (ie non-cancerous), preferably epithelial cells. Normal prostate epithelial cells exhibit number of characteristics for example, the cells are differentiated, have low motility and are non-invasive. Differentiated prostate epithelial cells express a number of characteristic cell markers for example, $CK_8$, PSA, PSMA, E cadherin.

In a further preferred method of the invention said epithelial cells are primary prostate epithelial cells.

Current methods which use cell-lines in monolayers typically use cell-lines isolated from one patient. This means any results produced from the cell-line EP not representative of the male population, nor the clonal nature of prostate cancer itself. It is currently believed that cancer treatments will have to be tailored to an individual's genetic profile. Therefore, cell-line models will have limited use in answering many questions required for clinical diagnosis and devising an appropriate treatment regime for cancer sufferers. Primary cultures provide a heterogeneous mixture of prostate epithelial cells and are derived from many patients thus providing a model which is representative of cell and patient populations, however, each primary cell line is specific to each patient.

In a further preferred method of the invention said prostate epithelial cells are cancerous.

Cancerous prostate epithelial cells are characterised by anchorage independent growth, an invasive and motile phenotype. Some cancerous epithelial cells are also characterised by an undifferentiated state which is reflected in the lack of expression of cell markers typical of normal epithelial cells. Cancerous cells also express a number of unique cancer specific antigens, so called tumour rejection antigens.

In a further method of the invention there are provided prostate epithelial cells characterised in that said cells are genetically engineered by recombinant techniques.

For example, and not by way of limitation, pro-drug activating genes may be transfected into prostatic cells to monitor the efficacy of pro-drugs as cytotoxic agents. A pro-drug activating gene refers to a gene the expression of which results in the production of protein capable of converting a non-therapeutic compound into a therapeutic compound, which renders the cell susceptible to killing by external factors or causes a toxic condition in the cell. An example of a prodrug activating gene is the cytosine deaminase gene. Cytosine deaminase converts 5-fluorocytosine to 5-fluorouracil, a potent antitumor agent. The lysis of the tumor cell provides a localized burst of cytosine deaminase capable of converting 5FC to 5FU at the localized point of the tumor resulting in the killing of many surrounding tumor cells. This results in the killing of a large number of tumor cells without the necessity of infecting these cells with a vector (the so-called "bystander effect"). Another example of a prodrug-activating gene is thymidine kinase (TK) (see US5,631,236 and US 5,601,818) in which the cells expressing the TK gene product are susceptible to selective killing by the administration of gancyclovir. This is merely meant to be illustrative of recombinant methods which could be used in combination with the cells according to the invention. Other examples may include the transfection of tumour suppressor genes, (eg p53). The term tumor suppressor gene refers to a nucleotide sequence, the expression of which in a target cell is capable of suppressing the cancerous phenotype and/or inducing apoptosis.

Genetically engineered prostate epithelial cells may be normal primary cells, cancerous primary cells, cloned normal cells or cloned cancerous cells.

In a further preferred embodiment of the invention said prostate epithelial cells are transformed with an oncogene, preferably a viral oncogene (e.g. the HPV E6 or E7 oncogenes, SV40 T antigen).

Methods to introduce nucleic acid into cells are well known in the art and typically involve the use of chemical reagents, cationic lipids or physical methods. Chemical methods which facilitate the uptake of DNA by cells include the use of DEAE -Dextran (Vaheri and Pagano Science 175: p434) . DEAE-dextran is a negatively charged cation which associates and introduces the DNA into cells but which can result in loss of cell viability. Calcium phosphate is also a commonly used chemical agent which when co-precipitated with DNA introduces the DNA into cells (Graham et al Virology (1973) 52: p456).

The use of cationic lipids (eg liposomes, see Felgner (1987) Proc.Natl.Acad.Sci USA, 84:p7413) has become a common method since it does not have the degree of toxicity shown by the above described chemical methods. The cationic head of the lipid associates with the negatively charged nucleic acid backbone of the DNA to be introduced. The lipid/DNA complex associates with the cell membrane and fuses with the cell to introduce the associated DNA into the cell. Liposome mediated DNA transfer has several advantages over existing methods. For example, cells which are recalcitrant to traditional chemical methods are more easily transfected using liposome mediated transfer.

More recently still, physical methods to introduce DNA have become effective means to reproducibly transfect cells. Direct microinjection is one such method which can deliver DNA directly to the nucleus of a cell (Capecchi (1980) Cell, 22:p479). This allows the analysis of single cell transfectants. Electroporation is arguably the most popular method to transfect DNA. The method involves the use of a high voltage electrical charge to momentarily permeabilise cell membranes making them permeable to macromolecular complexes. However physical methods to introduce DNA do result in considerable loss of cell viability due to intracellular damage. These methods therefore require expensive optimisation and also require expensive equipment.

More recently still a method termed immunoporation has become a recognised techinque for the introduction of nucleic acid into cells, (see Bildirici et al, Nature 405, 769) The technique involves the use of beads coated with an antibody to a specific receptor. The transfection mixture includes nucleic acid, typically vector DNA, antibody coated beads and cells expressing a specific cell surface receptor. The coated, beads bind the cell surface receptor and when a shear force is applied to the cells the beads are stripped from the cell surface. During bead removal a transient hole is created through which nucleic acid and/or other biological molecules can enter. Transfection efficiency of between 40-50% is achievable depending on the nucleic acid used.

In a further preferred method of the invention said cell culture support is collagen based.

In a yet further referred embodiment the serum its provided at between about 0.5%- 4% (v/v). Preferably said serum is provided at about between 1%-3% (v/v). Most preferably said serum is provided at about 2% (v/v).

According to a further aspect of the invention there is provided a cell culture composition comprising a collagen based cell support; stroma, oestrogen and dihydrotestosterone, wherein oestrogen is provided at about 10ng/ml and dihydrotestosterone at about $10^{-7}$M.

According to a further aspect of the invention there is provided a cancerous prostate like-acinus formed by the method according to the invention.

According to a yet further aspect of the invention there is provided a cancerous prostate like-acinus s been genetically modified by recombinant technique.

According to a yet further aspect of the invention, there is provided a cancer cell or cell-tine derived from the cancerous prostate acinus formed by the method of the invention. The cell or cell-lines may be genetically engineered.

According to a further aspect of the invention there is provided a method to identify agents capable of inhibiting the proliferation of cancerous prostatic cells comprising:

i) providing culture conditions and at least one cancerous acinus according to the invention;
ii) adding at least one agent to be tested; and
iii) monitoring the anti-proliferative activity of the agent with respect to the cells comprising the cancerous acinus.

According to a yet further aspect of the invention there is provided a method to identify agents capable of inhibiting the motility of cancerous prostatic cells comprising:

i) providing culture conditions and at least one cancerous acinus according to the invention;
ii) adding at least one agent to be tested; and
iii) monitoring the motility of cells comprising the cancerous acinus.

According to a further aspect of the invention there is provided a method to identify markers of prostate cell differentiation.

According to a further aspect of the invention there is provided a method to identify markers of prostate cell transformation.

Methods used in the identification of cell differentiation markers and/or markers of prostate cell transformation included based techniques (eg using the cells as complex immunogens to develop antisera to cell surface markers and the line) nucleic acid based techniques (eg differential screeing using cDNA from normal and transformed acini).

Also, it has been known for many years that tumour cells produce a number of tumour cell specific antigens, some of which are presented at the tumour cell surface. These are generally referred to as rumour rejection antigens and are derived from larger polypeptides referred to as tumour rejection antigen precursors. Tumour rejection antigens are presented via HLA's to the immune system. The immune system recognises these molecules as foreign and naturally selects and destroys cells expressing these antigens. If a transformed cell escapes detection and becomes established a tumour develops, Vaccines have been developed based on dominant tumour rejection antigens to provide individuals with a preformed defence to the establishment of a tumour. The method according to the invention provides a means to identify tumour rejection antigens and precursors which will have utility with respect to the vaccine development to provoke the patients own immune system to deter the establishment of prostate tumours.

According to a yet further aspect of the invention there is provided an *in vitro* method to analyse the development of cancerous prostatic cells from normal prostatic cells comprising transforming acini formed by the method according to the invention with an encogene capable of inducing prostatic cell transformation.

In a preferred method of the invention said normal prostatic cells are transformed with a viral oncogene.

It is well known in the art that there are agents capable of transforming a normal cell into a transformed cell with many of the features of cancerous cells. These include, by example only, viruses, DNA intercalating agents, oncogenes, telomerase genes. An embodiment described in the present application is the introduction of the E6 gene using retroviruses (amphotrophic).

An embodiment of the invention will now be described by example, only and with reference to the following figures;

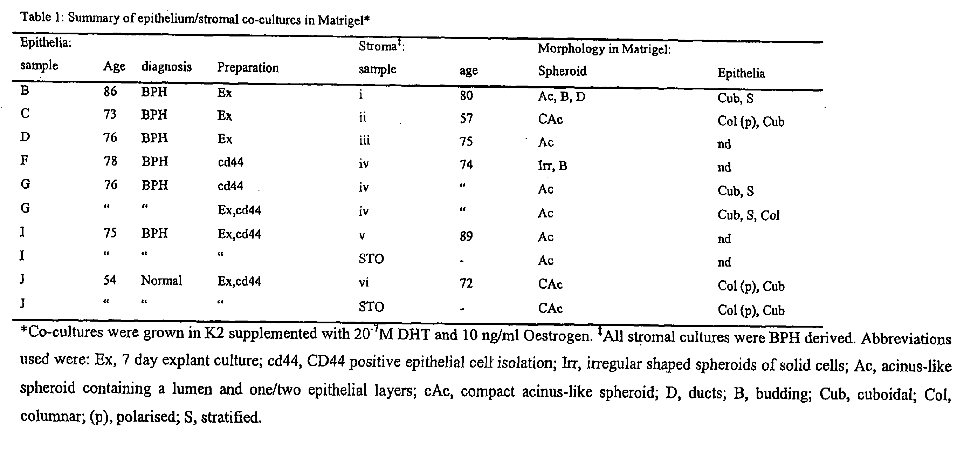

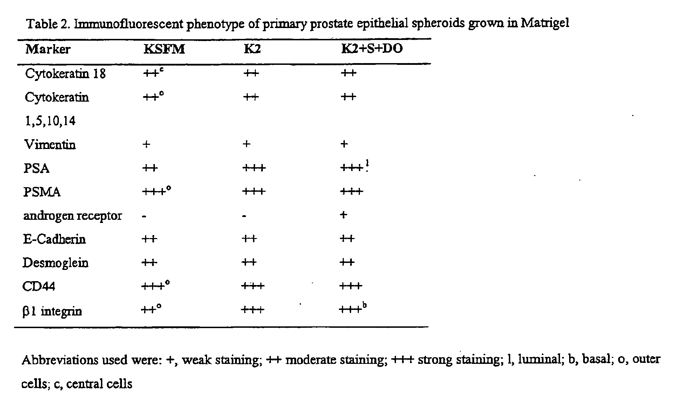

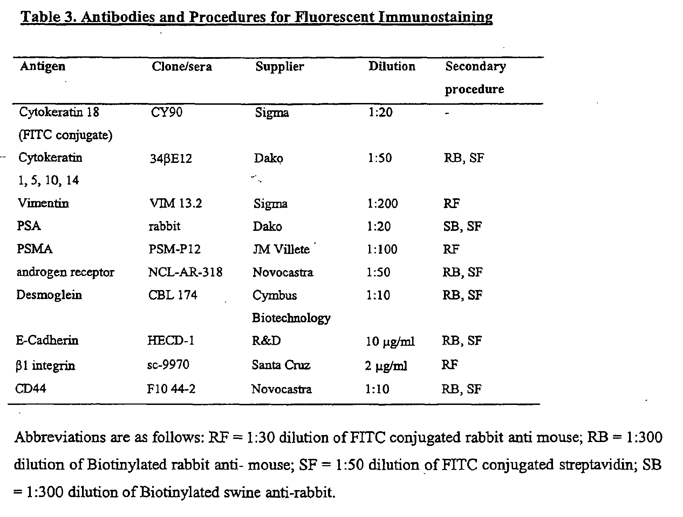

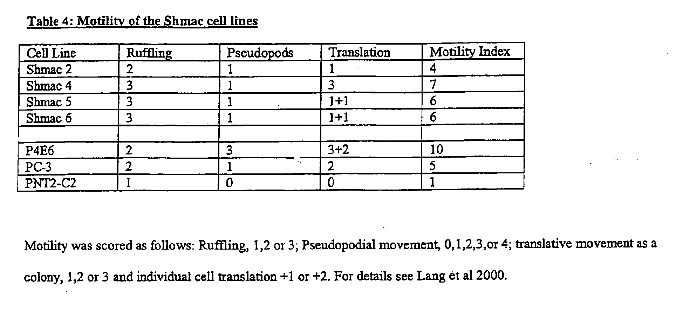

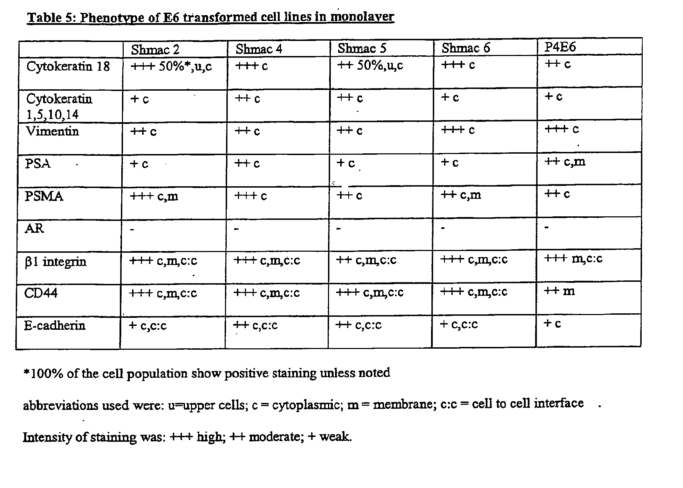

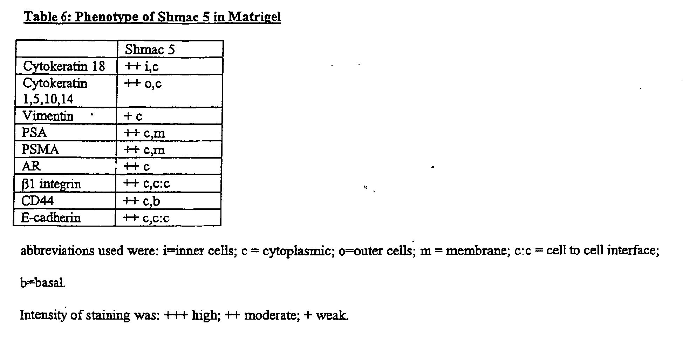

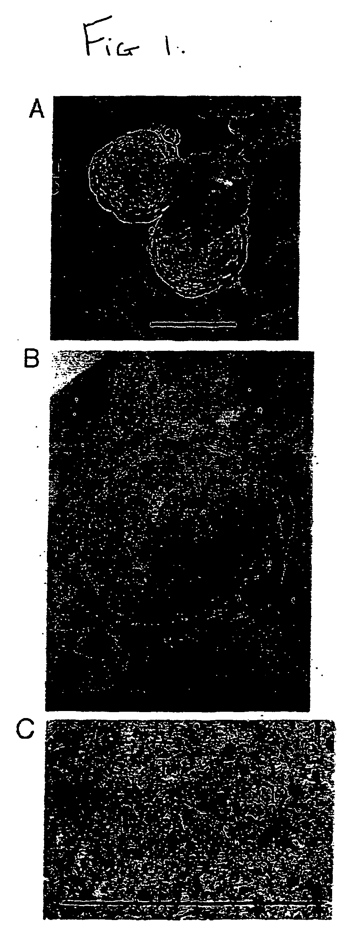

Figure 1. illustrates prostate epithelial spheroids grown in Matrigel and KSFM, (sample C). a) Phase image. Bar indicates 80 $\mu$m. b) TEM of a whole spheroid, bar indicates 10 $\mu$m. c) High magnification TEM indicating both tight (TJ) and desmosomal-like (D) junctions present between the tightly associating inner cells. Bar indicates 1 $\mu$m;

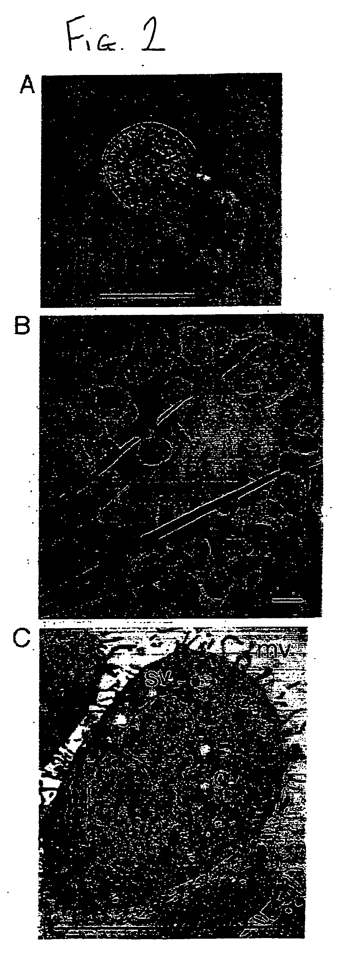

Figure 2. illustrates prostate epithelial spheroids grown in Matrigel and K2 (sample C). a) Phase image. Bar indicates 90 $\mu$m b) TEM of a whole spheroid, bar indicates 10 $\mu$m, c) High magnification TEM of a whole cell within the spheroid, showing luminal microvilli (mv), secretory vesicles (sv) and Golgi apparatus (G). Bar indicates 10 $\mu$m;

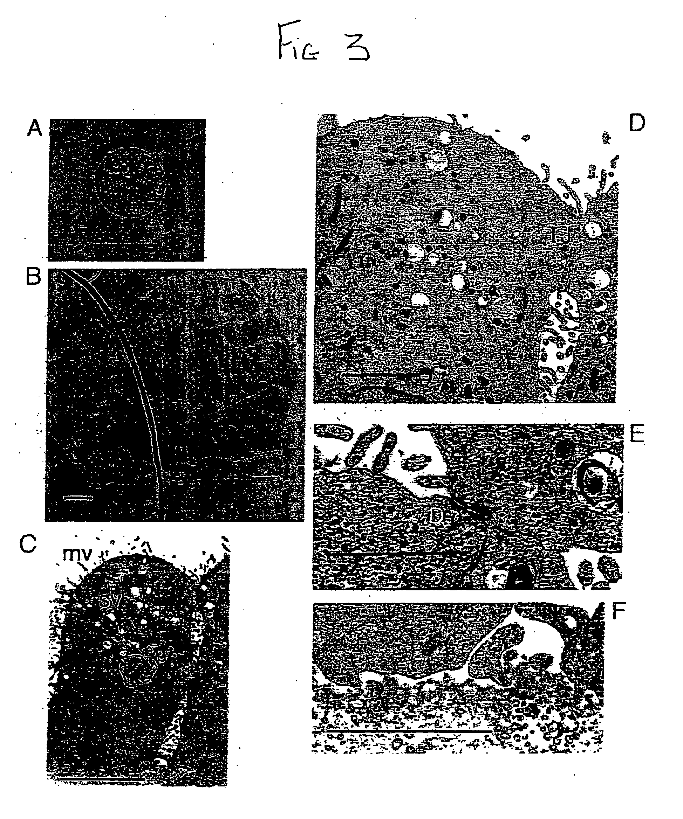

Figure 3. illustrates prostate epithelial spheroids grown in Matrigel, K2, $10^{-7}$ M DHT, 10 ng/ml OES and stromal (sample C). a) Phase image. Bar indicates 70 $\mu$m. b) TEM of whole spheroid, bar indicates 10 $\mu$m. c) High magnification TEM of a whole cell within the spheroid. Secretory vesicles (sv) are all polarised towards the lumen and microvilli are also visible on the huminal surface. Bar indicates 6 $\mu$m. d) High magnification TEM showing the luminal half of an epithelium (shown in c). The figure shows a large active golgi (g) and stacked rough endoplasmic reticulum. In addition atight junction (TJ) is visible at the luminal surface. Bar indicates 2 $\mu$m. e) A desmosomal-like junctional complex (D) present at a cell:cell interface on a luminal edge. Bar indicates 1 $\mu$m. f) Basal edge of spheroid showing that no intact basal lamina was visible. Bar indicates 1 $\mu$m;

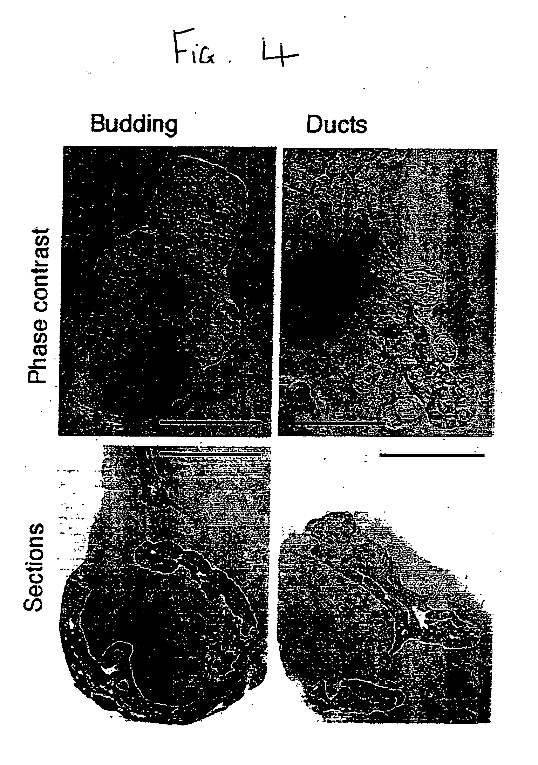

Figure 4. illustrates examples of a budding spheroid with multiple acini and duct-like structures also with evidence of budding, in phase contrast (bars indicate 100 $\mu$m). Toluidene blue stained thick sections of budding and duct-like structures, showing the presence of stratified cells (bars indicate 50 $\mu$m);

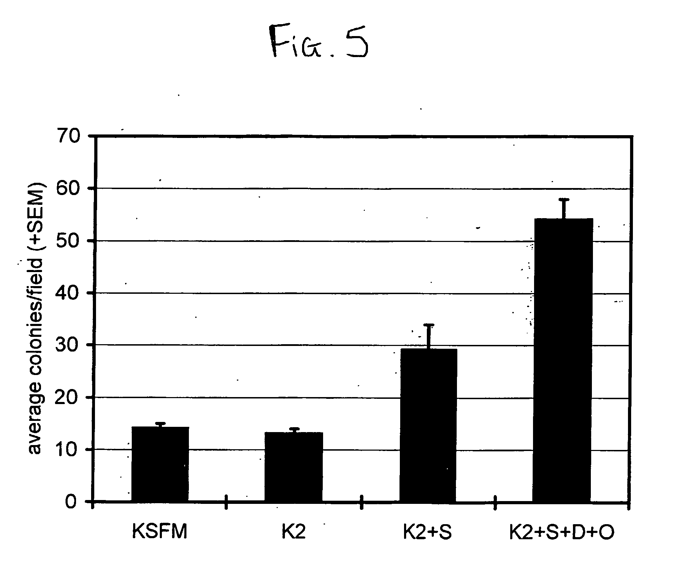

Figure 5. illustrates stromal cultures increased spheroid forming efficiency. Epithelial sample C was mixed into Matrigel and grown for two weeks in either KSFM, K2, K2 and primary stroma (S) or K2, S and $10^{-7}$ M dihydrotestosterone (D) and 10 ng/ml oestrogen (0);

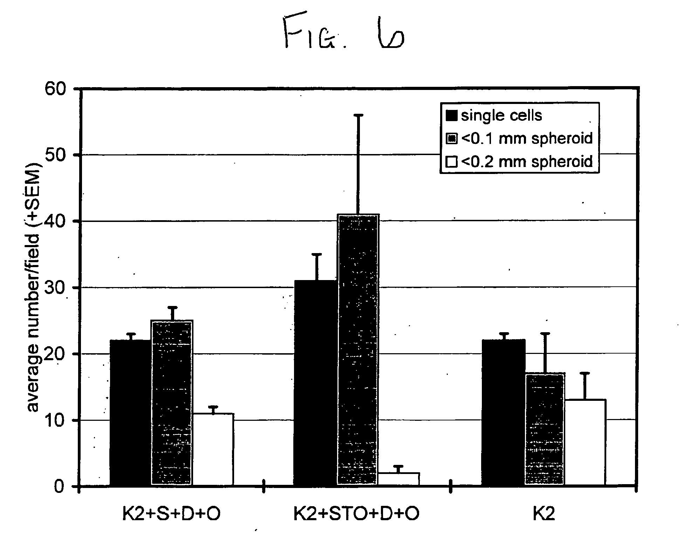

Figure 6. illustrates stromal cultures affect spheroid size. Epithelial sample J was mixed into Matrigel and grown for one week in either K2 or K2 plus primary stroma (S), $10^{-7}$ M dihydrotestosterone (D) and 10 ng/ml oestrogen (O) or K2 plus STO cells, D and O. Spheroid size was measures using a graticule;

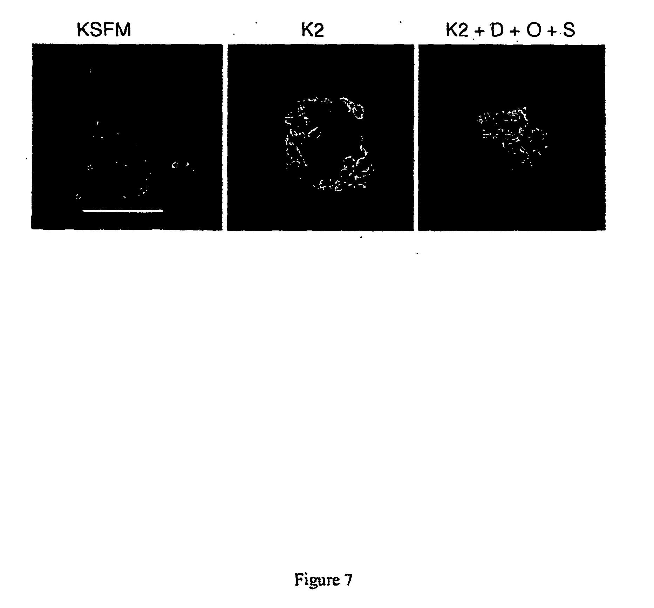

Figure 7. illustrates dual immunostaining of cytokeratin 18 (green) and cytokeratins 1, 5, 10, 14 (red) of prostatic epithelia grown in Matrigel. Epithelia (sample C) were grown in the presence of KSFM, K2 or K2 plus primary stroma

(S), $10^{-7}$ M dihydrotestosterone (D) and 10 ng/ml oestrogen (O), for 2 weeks. Cell nuclei in spheroids were counter stained with DAPI (blue). Bar indicates 80 $\mu$m.;

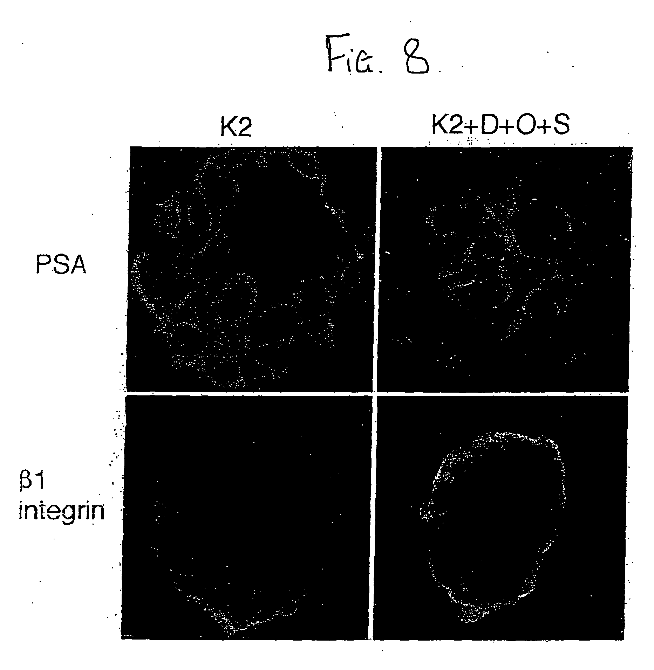

Figure 8. illustrates polarisation of PSA and $\beta$1 integrin in Matrigel epithelial spheroids when co-cultured with stroma. Using confocal analysis the expression of PSA and $\beta$1 integrin was compared between epithelial spheroids (sample C) grown in K2 or K2 plus primary stroma (S), $10^{-7}$ M dihydrotestosterone (D) and 10 ng/ml oestrogen (O), for 2 weeks. Bar indicates 70 $\mu$m;

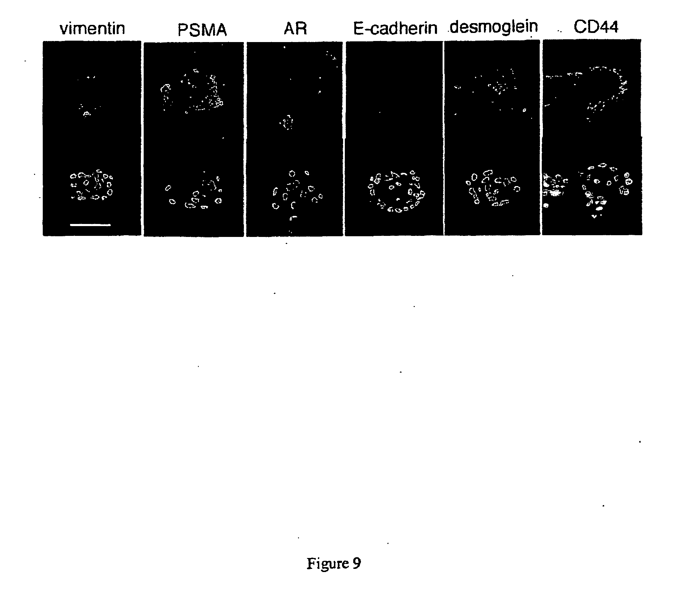

Figure 9. illustrates examples of immunohistochemical staining of prostatic epithelial matrigel spheroids. All spheroids shown were grown in KSFM, except for that illustrating androgen receptor expression which was cultured in the presence of K2, $10^{-7}$M dihydrotestosterone (DHT), 10 ng/ml oestrogen (O) and primary stroma. Spheroid nuclei were counter stained with DAPI (blue). (Epithelial sample C was cultured for 2 weeks). Bar indicates 80 $\mu$m;

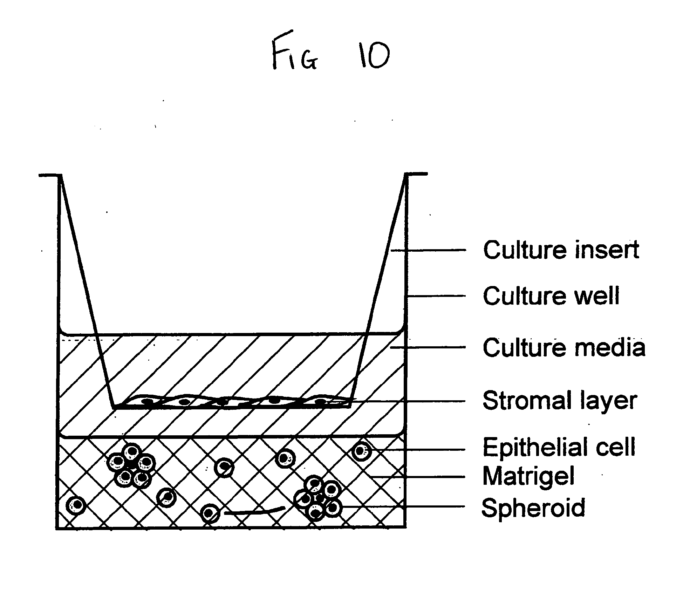

Figure 10. illustration of prostatic epithelial and stromal cell co-culture in Matrigel;

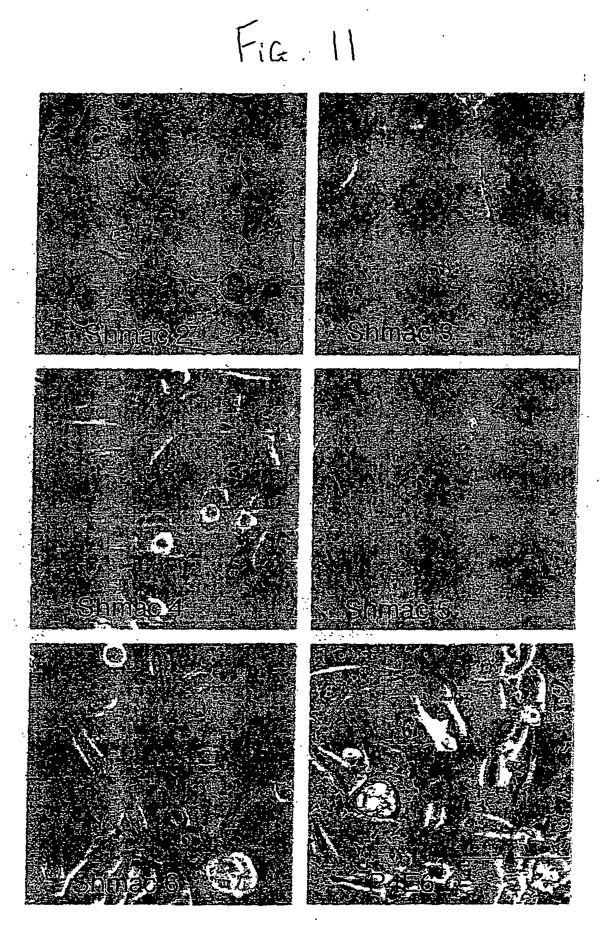

Figure 11 illustrates the morphology of Shmac cell lines and P4E6 growing as monolayers. Phase contrast pictures were taken at x10 objective magnification;

Figure 12 illustrates the growth curves of prostate cell lines growing in K2 medium in monolayer;

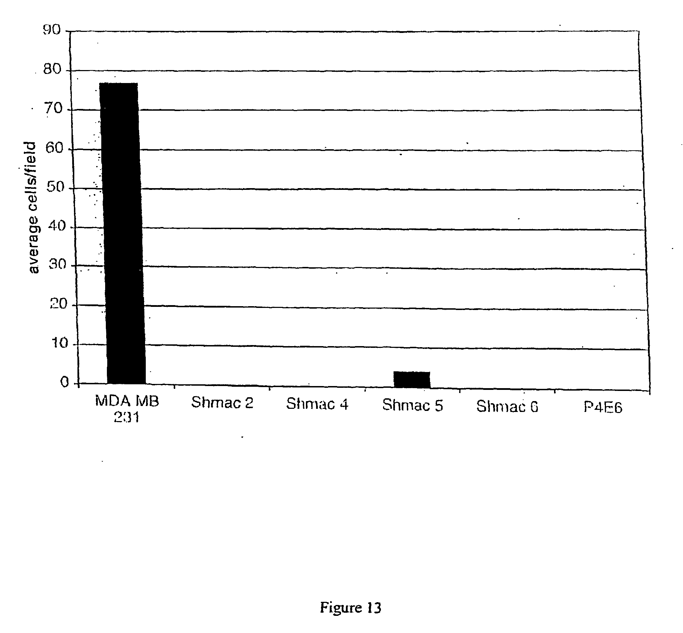

Figure 13 illustrates the invasive ability of Shmac cell lines through Matrigel coated cell inserts, in response to co-culture with stromal cell lines. Results are expressed as the mean of three triplicates;

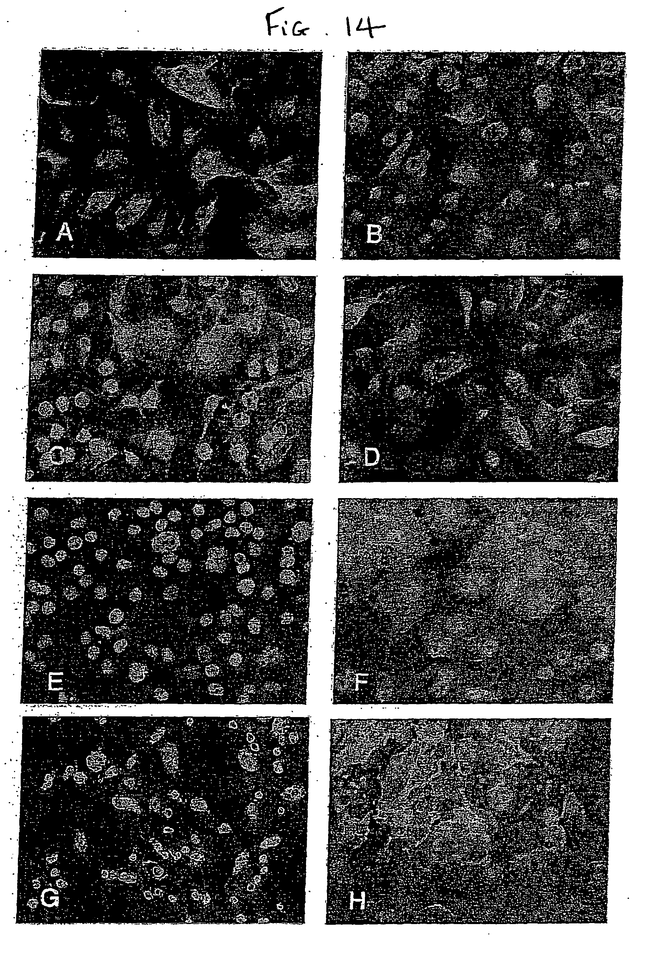

Figure 14 illustrates immunocytochemical staining of Shmac 5 cells growing in monolayer culture. A) Dual staining of cytokeratin 18 (red) and cytokeratins 1,5,10,14 (green). B) Vimentin. C) PSA. D) PSMA. E) Androgen receptor. F) E-cadherin. G) $\beta$1 integrin. H) CD44. Cell nuclei were counterstained with DAPI (blue). All images were captured at x20 objective magnification;

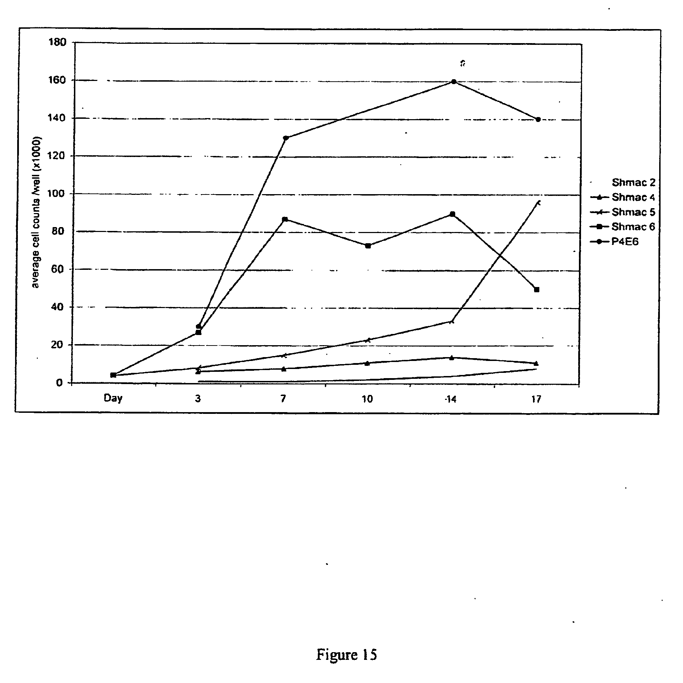

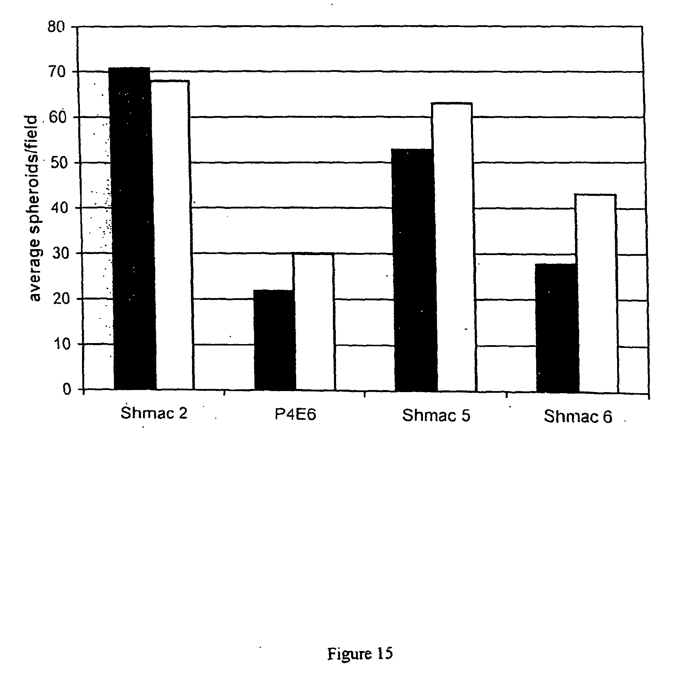

Figure 15: illustrates how stromal co-culture affects spheroids forming effciency. Epithelial cells were plated into Matrigel and grown for 1 week in K2 with (white) or without (black) stromal co-culture. The mean number of spheroids were counted per field. SE were less than 10% of the mean;

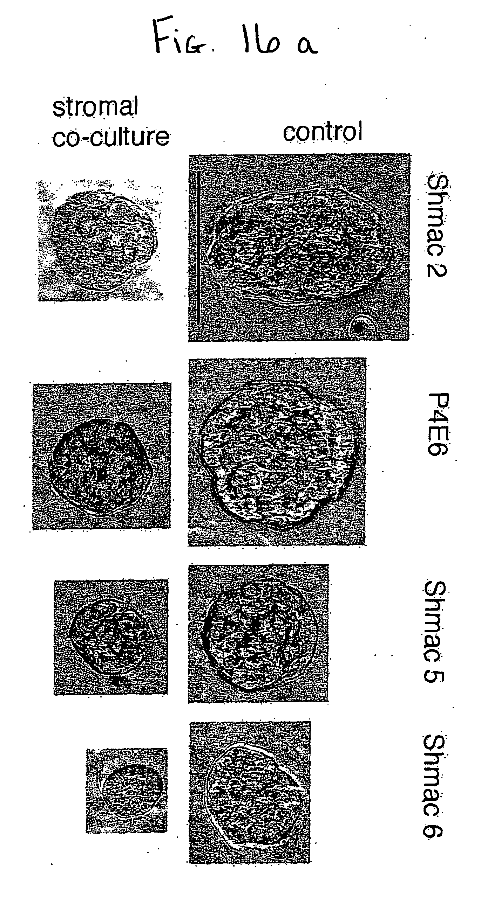

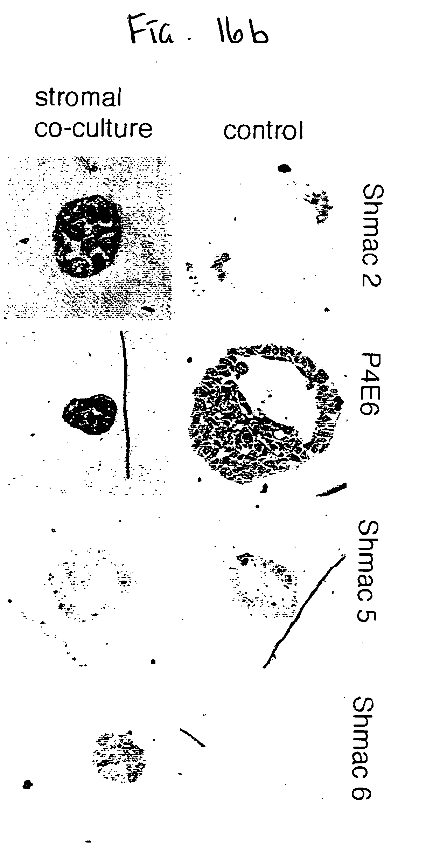

Figure 16 illustrates typical phase contrast morphologies (A) and 1 $\mu$m sections (B) of prostate cell line spheroids grown in Matrigel. All pictures were taken at x10 objective magnification after 7-10 days growth. Bar, 100 $\mu$m; and

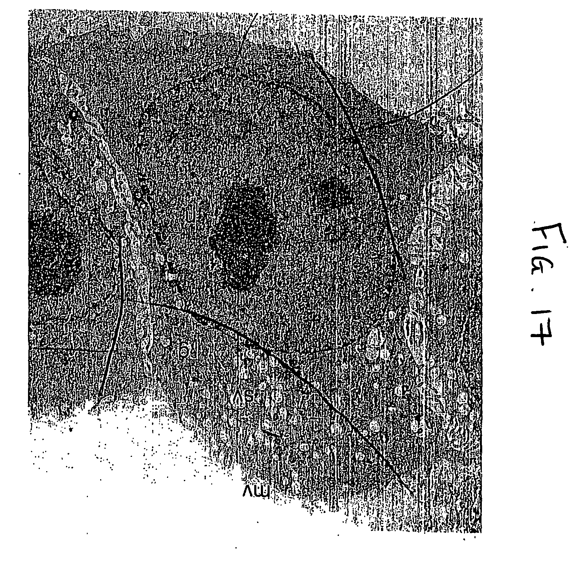

Figure 17 illustrates transmission electron microscopy of Shmac 5 epithelial cells grown in Matrigel in the presence of stroma. Cells are columnar in shape and show polarization of cellular organelles. Microvilli (mv), secretory vesicles (sv) and Golgi (G) were all luminal whilst the nucleus (n) was basal. Bar, 2 $\mu$m;

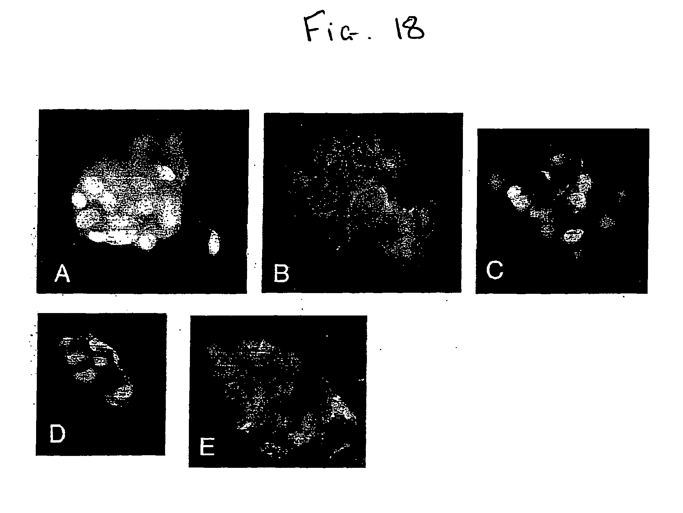

Figure 18 illustrates immunocytochemical staining of Shmac 5 cells growing in Matrigel culture. A) Dual staining of cytokeratin 18 (red) and cytokeratins 1,5,10,14 (green). B) PSA; C) Androgen receptor. D) CD44. E) $\beta$1 integrin. Cell nuclei were counterstained with DAPI (blue). All images were captured at x20 objective magnification;

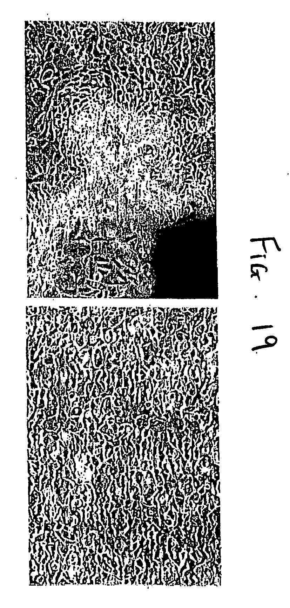

Figure 19 illustrates Comparative morphology of primary epithelial outgrowth and the E6 immortalised culture. Shown on the left panel is an epithelial outgrowth from a fragment of prostate tissue. The tissue is the large black object at the top right of the panel. In the right panel, the epithelia component from this outgrowth has been infected with a recombinant E6-expressing retrovirus and a cloned epithelial culture produced. Note the similar morphology;

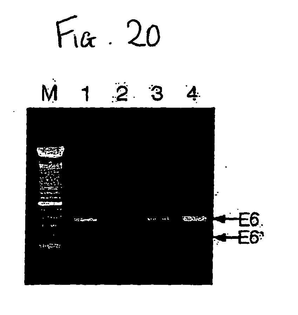

Figure 20 illustrates Detection of E6 DNA and mRNA in the immortalised cultures by RT-PCR Agarose gel electrophoresis of PCR products from an E6-transformed prostatic epithelial cell. Marker lane (M) is a 100 bp ladder from Life Technologies. Lane 1 is the amplification of cDNA from the cell line showing both E6 and E6*-specific products. Lane 2 is a negative control; lane 3 contains DNA from the same cell line (455 bp product only) and lane 4 is the CaSki cell DNA positive control; and

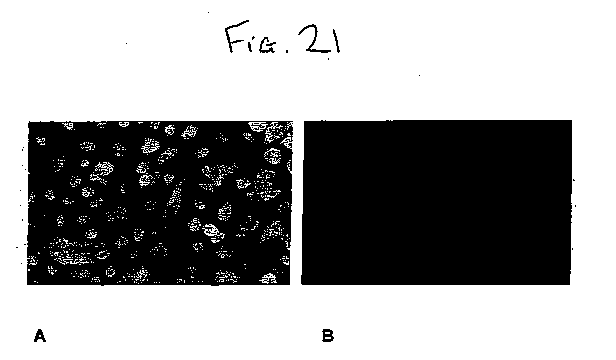

Figure 21 illustrates immunodetection of E6 protein in E6 transformed prostatic epithelial cells Panel A shows positive immunostaining (mainly pancellular) with an anti-E6 antisera (20) of the same cell line as analysed for DNA and RNA as shown in figure 2. Panel B is the corresponding negative control in which the primary antibody has been replaced with PBS in the full staining procedure;

## Materials and Methods

General chemicals were purchased from Sigma (Poole, UK), tissue culture media from Life Technologies (Paisley, UK) and tissue culture plastic from (Coming Costar Ltd., High Wycombe, UK) unless otherwise stated. Antibodies were purchased from Dako (High Wycombe, UK) unless stated.

## Cell Line Culture

STO cells (mouse embryonic fibroblasts) were obtained from the European Collection of Animal Cell Cultures (Porton Down, UK) and were routinely cultured in DMEM culture media (Life Technologies, Paisley, UK) supplemented with 10 % foetal calf serum (PAA Laboratories, GmbH, Linz, Austria) and 2 mM glutamine (Life Technologies). Cells were routinely cultured without antibiotics in a humidified atmosphere at 37 °C and 5% $CO_2$.

## Prostate Tissue Collection

Non-malignant tissue was obtained from consenting patients undergoing transurethral resection for benign prostatic hyperplasia or cystoprostatectomy for bladder cancer. 7 samples were collected for epithelial culture (age range 54-86) and 5 for stromal cultures (age range 57-89), summarised in table 1.

## Prostate Primary Cell Culture

Epithelial and stromal cultures were prepared (Lang, Clarke, George, Allen, & Testa. 1998) and characterised (Lang, Stower, & Maitland. 2000) as described before, these methods were based on those by Chaproniere and McKeehan (45). Briefly, prostatic tissue was digested by collagenase and trypsin, and differential centrifugation was used to enrich for epithelial and stromal fractions. The enriched stromal fraction was resuspended in stromal cell growth medium (RPMI 1640 medium supplemented with 10% FCS and 1% antibiotic/antimycotic solution) and cultured routinely in 75 ml tissue culture flasks. Stromal cultures were used between passages 2-5. The epithelial fraction was resuspended in keratinocyte serum free medium supplemented with 5 ng/ml epidermal growth factor, 50 $\mu$g/ml bovine pituitary extract and 1% antibiotic/antimycotic solution (media subsequently referred to as KSFM) and passed through a cell sieve (40 $\mu$m) to obtain single cells. Single cells were used immediately for further experiments, frozen for storage or plated into 25 ml flasks in 8 ml of KSFM and grown for 1 week.
Media conditioned by stroma was collected from confluent cultures of stromal cells by incubating the cultures for 48 hours in 15 ml of serum-free medium (DMEM/F12 supplemented with 10$\mu$g/ml insulin, 5$\mu$g/ml transferrin and 1 ng/ml selenium). Conditioned medium was removed, filtered (0.2 $\mu$m pore) and frozen at -20 °C until required.

## Cell Cultures and viruses

The PA317 murine packaging cell line was (ATCC CRL-9078) was obtained from the American Tissue Culture Collection. Retroviral transfer vectors pLNCX and pLXSN are can be obtained as part of the RetroX kit marketed by Clontech.

## Setting up primary prostate epithelial cultures for retroviral transduction

This method has been optimised for prostatic epithelium, but any method of tissue disaggregation can be employed. A critical step for the amphotrophic retroviral procedure is to obtain *dividing* cell cultures, as the viral life cycle is not completed in G0 cells (in this case a lentiviral vector could be substituted).

## Preparation of Biopsy for Culture

Tissue is mechanically disaggregated (chopped) in a sterile petri dish to produce pieces 1 mm$^2$ in diameter in 1 ml of transport medium (RPMI 1640, 3% (v/v) horse serum, 50 $\mu$gmr$^{-1}$ gentamycin (Sigma), 2.5 $\mu$gml$^{-1}$ Fungizone ).

## Seeding of Biopsy Material in Explant Culture

Using a disposable transfer pipette, the disaggregated biopsy is aspirated and transferred to 25 cm$^2$ tissue culture flasks with 0.2 $\mu$m vented lids (Coming). The medium of choice for the primary culture is described elsewhere (Primary Culture medium). The majority of the disaggregated tissue specimens should be seeded directly onto tissue culture plastic, but other substrata such as polylysine and collagen can be used to aid adhesion of the explants.

**Maintenance and growth of recombinant amphotrophic retroviruses.**

To generate producer cell lines for retrovirus, the recombinant DNA transfer vector must be transfected into the packaging cell line (PA317). This requires the insertion of the immortalising gene into a transfer plasmid vector, manipulated in bacteria, which mimics the proviral form of the retrovirus in its most primitive form i.e. a transgene coding region, flanked by the viral LTR sequences. Many such transfer vectors exist, and the immortalising gene used in this example is inserted in pLXSN (Genbank accession number M28248). This vector also contains an SV40 promoter-driven neomycin/G418 resistance gene to allow selection of the producer cells. The immortalising gene (the E6 gene from human papillomavirus) is under the control of the retroviral promoter in the LTR. A more elegant (and ultimately safer alternative) is to use the related pLNCX transfer vector (Genbank accession number M28247) in which the retroviral promoter in the LTR is inactive and the immortalising gene is under the control of a separate but stronger cytomegalovirus immediate early promoter. Complex kits for the generation and manipulation of amphotrophic retroviruses are now available commercially (Retro-X from Clontech).

**Maintenance of Retroviral Producer Cell Line**

The murine fibroblast cell line PA317 is one of several effective hosts for recombinant retroviruses. It contains the gag, pol and env open reading frames from the transfer vector pMAM3 co-transfected into 3T3 cells with an HSV1TK gene. The gag, pol and env genes are constitutively expressed and provide the "packaging" function for any small RNA (<9kb) with appropriate packaging signals derived from the retroviral terminal LTR sequences, such as pLXSN.

1. The cell line is maintained in D10 medium and is subcultured 1:10-1:40 every 4 -5 days, using standard techniques.
2. Replicate frozen stocks of PA317 are prepared in 40% DMEM, 50% FCS and 10% DMSO and stored in liquid nitrogen.

**Generation and Storage of Recombinant Amphotropic Retroviruses**

All transfections into PA317 are performed utilising Dosper® transfection (Roche) reagent.

1. Adherent cells are passaged 1:2 48 hours before transfection, and again, 24 hours before transfection at 50 - 80% confluency.
2. One microgram of transfer plasmid DNA is mixed with 3.125µl of Dosper reagent and serum-free medium added to about 0.1ml. The reaction is incubated at 20 C for 15 minutes.
3. Immediately before transfection, 10 volumes of the appropriate complete medium are added to the transfection mixture and the complete mixture gently pipetted onto the cells.
4. Transfections are carried out at 37°C for 5 - 12 hours, after which the transfection mixture is replaced with fresh complete medium.
5. Transfected cells are incubated for 6 - 16 hours, after which the cells are washed once with PBS and 1 ml D10 medium per 25 cm$^2$ added for a further 24 - 48 hours.
6. Transiently produced retroviruses are collected by removing the growth medium and floating cells removed by 0.45 µm disc filtration (stupor®, Gelman Sciences).
7. The retrovirus-containing supernatant can be stored at 4°C for up to several months or frozen at -80°C without further modifications.

**Titration of the Virus Stock**

1. HaCaT cells (ATCC number from (18) ) are plated into a 6-well tissue culture plate at 25 - 30% confluency, and left to completely adhere for 12 - 24 hours.
2. The medium is replaced with 0.7 - 1 ml fresh DF10 containing 8 µg/ml hexadimethrine bromide (polybrene). 10 µl of serial diluted retroviral supernatant (1:1, 1:2,1:10, 1:100, 1:1000, 1:10000) is added to the prepared HaCaT cells and incubated for 4 - 12 hours at 37°C.
3. After the transduction is complete, the retroviral supernatant is removed, the cells are washed several times with PBS, and maintained for a further 48 hours in complete DF10 medium.
4. HaCaT cells are then incubated with selection medium containing 500 µg/ml G418® (pLNCX or pLXSN series. Selection is carried out for 10 - 20 days, changing medium every 3-4 days.
5. For visualisation of generated colonies the cells can be stained with Giemsa's staining solution (BDH): The medium is removed, cells washed once with PBS and 500 $\mu$l Giemsa's staining solution added to each well. Cells are incubated for 10 - 20 min and excess staining solution removed with several washed with tab water. The fixed cells are air-dried and colonies counted.

6. The titre is calculated as follows, and expressed in colony-forming units (cfu) per millilitre of virus (after (19)).

$$G418 - resistant\ CFU/ml = \frac{number\ of\ colonies}{retroviral\ dilution\ volume\ (ml)}$$

### Immortalisation of primary prostatic epithelium

The actual immortalisation procedure is an extension of protocol above, used to assay the recombinant viral stock. Prostatic epithelial cells are notoriously difficult to transfect by conventional precipitation or liposome mediated techniques, but our experience with retroviruses indicates that the cells are readily infectable with as high an efficiency as most mammalian cell lines.

### Infection of the Primary prostatic epithelial cells with retrovirus stock

For infection of primary prostatic epithelial cells undiluted viral stock is used.

1. The medium is drawn off from the prostatic cell outgrowths.
2. Cells are washed 2x5 min. in PBS prior to addition of 1.5 ml of virus with polybrene at 8 $\mu$gml$^{-1}$ per 25 cm$^2$ flask. Cells are incubated in the presence of the virus for 2 hours at 37°C, 5% CO2.
3. After this time, the polybrene-containing medium is removed and the cells washed 2x5 min. with PBS. This is replaced with fresh D10 medium, and the cells incubated for a further 48 hours prior to selection with G418 at 25 $\mu$gml$^{-1}$.

### Ring Cloning of Transfected Cells

After 10-14 days of drug selection discrete colonies are observed which can be individually ring cloned into 12.5 cm$^2$ flasks.

1. Cells are washed 2x3 min. in PBS-Ca$^{2+}$/Mg$^{2+}$, and the lid of the flask cut off under sterile conditions.
2. A sterile glass ring (10 mm in diameter) is dipped in autoclaved petroleum jelly and placed over the individual colonies, creating a seal.
3. 500 $\mu$l of 0.25% (v/v) trypsin/EDTA was placed in the ring and immediately aspirated off together with the PBS.
4. Trypsin is again applied and the cells monitored. As they began to round up, cells are gently pipetted up and down and placed in flasks containing R10 culture medium.

The isolated colonies are incubated at 37°C, 5% CO$_2$ until they can be subcultured into larger tissue culture flasks.

### Cell Lifts for DNA Purification

To monitor the immortalisation process, and to provide an indication of the origin of the cells immortalised, a micro-assay from the growing immortalised colonies of epithelial cells can be carried out. Using the procedure described below, sufficient cells are obtained to carry out a PCR amplification of either known genes (to compare mutation status between the original tumour and the cellular outgrowths) or a microsatellite/Single nucleotide polymorphism analysis. Full protocols for the latter analysis are available elsewhere.

1. The medium is aspirated from growing cells, which are then washed 2x5 min. in PBS.
2. Squares of 3MM paper are cut (measuring 3x3 mm) and sterilised by autoclaving in a glass petri dish.
3. Using sterile forceps, the 3MM squares are placed onto the cells, left for 20 sec. and then removed into an eppendorf tube containing 200 $\mu$l of DNA extraction buffer.
4. Fresh medium is restored to the cultured cells, which can then continue growing.
5. The 3MM squares are processed as follows. Fifty microlitres (or more) are added to an eppendorf tube containing the 3MM square and incubated overnight at 42 C.

6. Next day, the proteinase K was inactivated by incubation at 95 C for 8-10 minutes, and the paper either centrifuged to the bottom of the tube or carefully removed with a sterile tip.

7. The resulting solution is ready for PCR amplification and further analysis, when used to make up no more than 10% of a PCR reaction final volume.

## Detection of E6 DNA in Infected Cells

1. Cells are pelleted by centrifugation and the DNA extracted by standard methods. 20 ng of the DNA is used as a substrate for PCR.

2. Reaction mixtures contain 2 mM dNTPs, 0.05% W-1, 1.5 mM MgC12, 0.3 $\mu$M forward and reverse primers (see note 6) and 0.5 U of *Taq.* DNA polymerase. Thirty five cycles of amplification, with annealing at 55 C are sufficient to detect the low copy numbers of E6 retained in the immortalised cells.

3. PCR product (455bp) is detected by electrophoresis in a 1% (w/v) agarose gel.

## RT -PCR to detect E6 mRNA

1. 5 $\mu$g total cell RNA is pipetted into a DEPC-treated Eppendorf tube with 0.5 $\mu$l of RNAguard solution (Boeringer Mannheim), 500 ng of oligo-dT primer and sterile ddH$_2$O to a volume of 10.5 $\mu$l.

2. The tube is heated to 70°C for 10 min. and snap-cooled on ice. The Eppendorf tube is then pulsed in a microfuge to collect the contents.

3. The cDNA synthesis reaction is set up by the addition of 0.5 $\mu$l RNAguard, IX Superscript buffer (Boehringer Mannheim), 10 mM DTT, 1 mM dNTPs and 200 U of Superscript enzyme.

4. The contents of the tube are mixed gently and incubated at 42°C for 1 hr. After this period the cDNA is precipitated at -80°C for 1 hr. by addition of 0.05 volumes of glycogen solution, 0.5 volumes of 3 M NaCl and 3 volumes of absolute ethanol.

5. The cDNA is pelleted by centrifugation at 15,000 rpm at 4°C for 5 min. and following a final wash with ice-cold 70% ethanol the pellet is air-dried and resuspended in 20 $\mu$l sterile ddH$_2$O.

6. 2 $\mu$l of cDNA is used as a substrate for PCR to detect E6 expression. The reaction mixture also consists of 2 mM dNTPs, 0.05% W-1 detergent, 1.5 mM MgCl$_2$, 3 pmoles of each of the forward and reverse primers (see note 6), 1xPCR buffer and 1 U of *Taq.* DNA polymerase (Gibco-BRL).

7. Products are resolved by electrophoresis in a 1% (w/v) agarose gel as shown in Figure 20.

## Shmac Cell-Lines

The Shmac series of prostate epithelial cell lines were derived from a sequential series of tissue biopsies, grown as explants in primary culture as described above and infected with E6 retrovirus as described in Maitland *et al* (2001). Individual populations selected by drug (G418) resistance are not immortal (like P4E6) but have an extended life span.

## Immortalisation and culture of cell lines

Shmac 2, 3 and 6 cells were derived from benign prostatic hyperplasia. Shmac 4 cells were derived from a well differentiated tumour (1+2) and Shmac 5 from a moderately differentiated tumour (3+3). P4E6 was immortalised from prostate epithelial cells derived from a well differentiated tumour, Gleason score 4 [Maitland et al 2001]. Epithelial cell lines were routinely cultured in keratinocyte serum free media supplemented with 2 % foetal calf serum (PAA Laboratories, GmbH, Linz, Austria), 5 ng/ml epidermal growth factor and 25 $\mu$g/ml bovine pituitary extract (K2).

STO cells (mouse embryonic fibroblast cell line) were obtained from the European Collection of Animal Cell Cultures (Porton Down, UK) and were routinely cultured in DMEM culture media supplemented with 10 % foetal calf serum and 2 mM glutamine. All cells were routinely cultured without antibiotics in a humidified atmosphere at 37°C and 5% CO$_2$.

## Cell Morphology and Growth assay of Shmac Cell-Lines

Phase images were observed with a Nikon TE300 inverted microscope and captured with a JVC 3-CCD video camera. Images were subsequently prepared using Adobe Photoshop 4. For growth assays cells were prepared at a concentration of $10^4$ cells/ml in appropriate growth media. 200 μl of cell solution was then added to the well of a 96 well plate. Cells were media changed or counted every 3-4 days. Cell counts were performed by haemocytometer after trypsinisation. Cell solutions were diluted with trypan blue and viable cell counts taken.

## Invasion and motility assays for Shmac Cell-lines

Both assays were performed as detailed in Lang et al, 2000. Briefly, motility was measured by observing an epithelial cell colony of approximately 8-16 cells. Phase contrast images were captured every 4 minutes for 8 hours using a JVC video camera, and recorded on computer using a Scion Image CG7 frame grabber [Scion Corporation, Frederick, Maryland, USA]).

Motility was scored by assessing membrane ruffling, pseudopodial and translative movement, based on the method of Mohler et al, 1988.

Invasion was measured by counting the number of epithelial cells invading Matrigel (Becton Dickinson, Oxford, UK) coated cell culture inserts (8 μm pore, Becton Dickinson) in serum free medium. Inserts were placed in 24 well plates which contained confluent cultures of STO stromal cells. Epithelial invasion was measured overnight, after which the inserts were removed and crystal violet was used to stain and count the cells which had invaded to the underside of the insert.

## Isolation of CD44+ Epithelial Cells

Single cell suspensions of primary prostatic epithelia ($10^6$ cells) were labelled with 2.5 μg anti-CD44 (Pharmingen, Becton Dickinson UK Ltd., Oxford, UK) for 5 mins at 4°C and then washed extensively using PBS supplemented with 2mM EDTA and 0.5% (w/v) BSA. Antibody was then linked to 20 μl goat anti-mouse MACS microbeads (Miltenyi Biotec Ltd., Bisley, UK) at 4°C for 15 mins, the cells were again washed extensively after which they were added to a MACS column and the labelled basal cells were eluted and resuspended in appropriate culture media (basal cells formed 10-43% of the total epithelial cell population).

## Cell Culture in Matrige

Epithelial cells were prepared at a concentration of 60 000 cells/ml in KSFM. On ice they were mixed 1:1 *(v/v)* with Matrigel (Becton Dickinson, Oxford, UK) and 0.25 ml aliquots were subsequently plated into 24 well plates. The Matrigel was set by incubating at 37 °C for 30 mins. For experiments requiring stromal co-culture, stroma was pre-grown onto cell culture inserts, these were then placed on top of the Matrigel/epithelial cell mix (illustrated in figure 1). 1 ml of required growth media was added to each well and cells were thereafter medium changed every 3 days, by the removal of 0.5 ml of spent media and the addition of 0.5 ml of fresh media. Equivalent batches of Matrigel were used throughout. Phase images were observed with a Nikon TE300 inverted microscope and captured with a JVC 3-CCD video camera. Images were subsequently prepared using Adobe Photoshop 4.

## Transmission Electron Microscopy

Cells growing in Matrigel were washed twice with phosphate buffered saline (PBS) and then fixed for 1 hour at room temperature in 100 mM phosphate buffer, 4% paraformaldehyde (TAAB, UK) and 2.5% Ultrapure glutaraldehyde. Cells were further processed for electron microscopy as described by Allen and de Wynter (46). Thick sections were cut at 1 μm and stained with 0.6% toluidine blue in 0.3 % sodium bicarbonate. 70 nm sections were cut and stained with saturated uranyl acetate in 50% ethanol followed by Reynolds lead citrate and observed with a Jeol JEM 1200 Ex transmission electron microscope.

## Fluorescent Immunostaining

Cells grown in Matrigel were snap frozen in liquid nitrogen after embedding the gel in OCT Compound (BDH, Poole, UK). Embedded gels were stored at -20°C. 7 μm sections were cut on a Leica cryostat and mounted onto Super frost microscope slides (BDH).

Immunostaining was carried out according to table 2. Antibodies were prepared in PBS supplemented with 1% bovine serum albumin. Each step was followed by three washes in PBS. Primary antibodies were incubated at room temperature for one hour and secondary antibodies for 30 minutes. Spheroids were counter stained with 1 μg/ml DAPI. Coverslips were mounted to slides using Cityfluor (Agar Scientific Limited, Stansted, UK). Immunostained cultures were observed and photographed using a Nikon Eclipse TE300 fluorescent microscope. Digital images were subsequently prepared using Adobe Photoshop 4.

**Confocal Microscopy**

Samples embedded in OCT were sectioned at 20 μm and inmmunostained as described above. Sections were then observed at 1 μm layers using a MRC1000 Biorad Confocal Microscope (Hemel Hempstead, UK).

**EXAMPLE 1**

Seven day cultures of non-malignant prostatic epithelia were seeded as single cells directly into Matrigel, and in the presence of KSFM, the single cells developed into spheroids which were irregular in shape (figure 1a). TEM demonstrated that these spheroids were solid masses of both cuboidal and stratified cells and their appearance was consistent with a hyperplastic growth (figure 1b). In the centre of the spheroid there was evidence of necrosis. The central stratified/cuboidal cells had very tight cell to cell contacts (figure 1c) whereas the outer cuboidal cells contacted each other much more loosely, had relatively sparse cytoplasms and elongated nuclei with prominent nucleoli. High power magnification indicated there were multiple junctional complexes consistent with desmosomal-like and tight junction-like cell contacts, present between both cell types (examples shown in figure 1c). The presence of oestrogen, dihydrotestosterone or media conditioned by prostatic stromal cultures did not affect the morphology (results not shown).

Addition of 2% serum to the media led to the spheroids appearing less dense (figure 2a), TEM indicated this was due to the spheroids developing lumen (figure 2b). The spheroids had 1/2 epithelial cell layers and were cuboidal or columnar in shape. Microvilli were observed at the luminal edge of the epithelium but other signs of polarisation were not evident. Golgi bodies, secretory vesicles and stacked rough endoplasmic reticulum (RER) were all present, consistent with a secretory function. No basal lamina was observed and few junctional complexes were observed. Serum was included in these experiments to support stromal growth.

Research has shown that stroma, oestrogen and dihydrotestosterone are required to induce prostate epithelial differentiation (Bayne, Donnelly, Chapman, Bollina, Buck, & Habib. 1998)). The addition of these factors plus serum to epithelium growing in Matrigel led to the formation of compact spheroids which were regular in shape (figure 3a). TEM demonstrated the spheroids were similar to *in vivo* acini since they contained lumen surrounded by one or two epithelial cell layers which were closely organised and columnar (figure 3b). Higher magnification (figure 3c) indicated the cells were polarised, such that microvilli, golgi and secretory vesicles were organised to the luminal side whilst nuclei were predominantly basal. Golgi were consistently large and stacked RER was evident. No intact basal lamina was visible though a greater number of junctional complexes (desmosome-like and tight junction-like) were visible laterally and were predominantly toward the lumen.

This experiment was carried out in parallel on three different epithelial cultures (B, C, D). The results were similar for all, however only sample C (shown in figures 1-3) demonstrated a high degree of polarisation in the presence of stroma (figure 3b). Samples B and C produced lumen containing spheroids with columnar or cuboidal epithelium but no polarisation.

Table 1 summarises all repeat experiments in K2, DHT, Oes and stroma. Overall experiments in these culture conditions showed evidence of columnar polarised epithelia in 2/4 examined epithelial samples (C, J). In two separate experiments spheroids did not grow in serum free conditions (samples F and G). Growth in 2% serum consistently led to the formation of spheroids with lumen (5/7 samples) where it did not there was no growth (sample F) or there was irregular spheroid formation (sample G), as illustrated in figure 1. The sample showing no growth, produced spheroids only in the presence of stroma and in this instance the spheroids were irregular. The sample which produced irregular spheroids in 2% serum, produced lumen in the presence of stroma but the epithelia were cuboidal, columnar and stratified, with no evidence of polarisation. Subsequently, TEM analysis of polarisation was only carried out if compact acinus-like spheroids were observed.

Basal epithelium in the prostate express CD44 (25) and may represent a candidate epithelial population more likely to differentiate in Matrigel culture. Therefore, we selected CD44 positive epithelium from four (of seven) prostate epithelial preparations (F, G, I, J). No noticeable differences were observed in spheroid formation or morphology in comparison to those produced from whole epithelial populations (samples B, C, D). However, CD44 negative epithelial populations showed no growth within Matrigel (results not shown).

Two of the samples (B, F) formed budding and ductal structures when grown in K2, DHT, OES and stroma, a further two (D, G) also exhibited such morphologies when grown without stroma (examples shown in figure 4). All samples which produced budding and ductal structures were accompanied by stratified epithelia. Since epithelia are not normally stratified in prostatic duct or acini we concentrated our studies on the acinus-like spheroids.

## EXAMPLE 2

The presence of stromal cultures was found to significantly increase the spheroid forming efficiency of epithelial samples. Figure 5 indicates that approximately equal numbers of spheroids formed in KSFM and K2 (sample C). In the presence of stroma, the number of spheroids formed approximately doubled, and further increased with the addition of oestrogen and dihydrotestosterone. Two other samples (B and D) examined in parallel, showed increased spheroid formation only in the presence of stroma (approximately double), but hormones had no further effects. Increased spheroid formation in the presence of stroma was reproduced on three further samples examined on separate occasions (F, G, J). In addition, presentation of the stroma in the co-culture was examined by comparing stroma within an insert to that directly mixed with epithelia in the Matrigel, or added to the top of a preset gel. Our results indicated that stroma co-cultured within an insert produced maximal spheroid formation. In addition, the different ways of presenting stroma had no effect on spheroid morphology (results not shown). We also observed that the use of epithelial cells from 7 day explants rather than freshly isolated led to greater spheroid forming efficiency and that the spheroids subsequently produced maintained in culture for longer periods (results not shown).

## EXAMPLE 3

The size and type (irregular or acinus-like) of epithelial spheroids forming within Matrigel varied between samples (summarised in table 1). However we consistently observed that stromal co-cultures predominantly produced smaller sized spheroids. Figure 6 shows that, after 1 week in Matrigel and K2, equivalent numbers of 0.1 mm and 0.2 mm diameter epithelial spheroids had grown (22 cells/field did not form spheroids but remained as single cells). In total, an average of 30 spheroids/ field formed. In the presence of primary and cell line stroma 36 and 43 total average spheroids/ field, formed respectively, but were predominantly 0.1 mm in diameter. Notably, co-culture with STO cells led to greater numbers of spheroids forming.

## EXAMPLE 4

Spheroids were sectioned and stained by fluorescence immunohistochemistry to compare the phenotypic profiles of those grown in serum free conditions to those grown with sera, stroma and hormones. The spheroids were phenotyped by investigating a variety of differentiation markers. Luminal prostatic epithelium were identified using; cytokeratin 18 and prostate specific antigen (PSA) (Nagle. 1996)), whilst basal epithelial cells were identified using; basal cytokeratin (1,5,10,14), CD44 and $\beta$1 integrin (Knox, Cress, Clark, et al. 1994)). Vimentin was analysed since it can reflect differentiation (Iwatsuki, Sasaki, Suda, & Itano. 1999). Androgen receptor, PSA and prostate specific membrane antigen (PSMA) served as functional differentiation markers (30, 31). Finally, the cell adhesion molecules, E-cadherin and desmoglein were also analysed. The results are summarised in table 2 and examples of each stain are shown in figures 7, 8, and 9. Intermediate filaments stained at similar intensities between the different spheroid types (table 2). However, localisation of cytokeratins 18 and 1,5,10,14 varied between the different spheroids (figure 7). Spheroids grown in the presence of KSFM showed expression of cytokeratins 1,5,10,14 in the epithelia at the outer edge of the spheroid, whilst cytokeratin 18 was expressed independently by the cells in the middle of the spheroid. Spheroids grown in the presence of serum and/or stroma were predominantly cytokeratin 18 positive but also co-localisation of cytokeratins 18 and 1,5,10,14 was observed. PSA was strongly expressed in all the spheroids, but, expression was polarised (towards the lumen) in spheroids grown in the presence of stroma (figure 8). PSMA was strongly expressed by all spheroid types, but expression was stronger in the outer cells of spheroids grown in serum free conditions (figure 9). Androgen receptor was only weakly detected in spheroids grown with stroma (figure 9). E cadherin and desmoglein were expressed by all spheroids at cell to cell contacts. CD44 and $\beta$1 integrin were likewise strongly expressed by all spheroids at the cell membrane, but noticeably both markers were only expressed by the outer cells of spheroids grown in serum free conditions. In addition, $\beta$1 integrin expression was strongly polarised (basally) in the presence of stroma (figure 8).

The present study demonstrates for the first time that human primary prostate epithelium seeded into Matrigel can form acinus-like structures in the presence of stroma, androgen, oestrogen and serum. These acini show a high degree of functional (PSA+/PSMA+/androgen receptor+) and morphological differentiation consistent with human prostatic acini *in vivo.* This represents a very useful model with which to study prostatic biology and will complement existing animal models (Hayward, Rosen, & Cunha. 1997)) by reducing the complexity inherent in such systems.

PSA expression can be induced by Matrigel alone (shown here) or stroma alone (Bayne, Donnelly, Chapman, Bollina, Buck, & Habib. 1998)). Induction of PSA expression by epithelium without stroma indicates that epithelial differentiation is partly inherent. In our model, both Matrigel and stroma were clearly required to induce architectural organisation, androgen receptor expression and polarised secretion of PSA. Previously, androgen receptor expression in human primary prostate has been observed in both epithelia and stroma when co-cultured together but not in isolation (Bayne, Donnelly, Chapman, Bollina, Buck, & Habib. 1998), emphasising the importance of both cell types for terminal epithelial differentiation. The requirement for stroma to induce the correct architectural organisation has previously been demonstrated in mouse models (32). Our results also found that stromal co-culture produced greater numbers of small spheroids, which may indicate that the stroma and hormones either reduced growth or increased adhesion (thereby compacting the cells into a smaller spheroid). Stroma was clearly important for increasing spheroid forming efficiency. The ability of stroma to double spheroid forming efficiency suggests that stroma can recruit more epithelia to form spheroids. It is possible that epithelium in isolation can form spheroids if they have already received signals to differentiate but are then unable to undergo proper differentiation. The factors governing these differentiation pathways are unknown. One stromal derived factor, hepatocyte growth factor was found to increase the growth of primary lung epithelium and also increase the numbers of spheroids formed in Matrigel two-fold (Sato & Takahashi. 1997). Hepatocyte growth factor is clearly worth further investigation in our own model system.

The addition of unknown serum factors to Matrigel went some way to producing the correct morphological organisation of spheroids into acinus-like structures, but stromal co-culture was required for greater differentiation. Experiments examining the behaviour of primary mammary epithelium cultured in Matrigel alone found that breast specific proteins can also be expressed (Chen & Bissell. 1989)5). However, breast epithelial spheroids can be grown in Matrigel and demonstrate both functional and morphological differentiation in the presence of serum and hormones alone (Barcellos-Hoff, Aggeler, Ram, & Bissell. 1989). Stromal coculture systems increased differentiation, and, in agreement with our own results, produced alveolar morphogenesis rather that ductal (Darcy, Zangani, Shea-Eaton, et al. 2000). Breast studies have shown that differences in growth factor/ kinase receptor activation can account for alveolar or ductal morphologies (Niemann, Brinkmann, Spitzer, et al. 1998). Recent studies indicate that a complex mix of growth factors and hormones can override the need for serum when breast epithelium is grown in Matrigel with stromal co-culture (Darcy, Zangani, Shea-Eaton, et al. 2000), and such studies are clearly now required to understand the important factors in prostatic differentiation. Stromal co-culture is also required for the induction of functional and morphological differentiation in other organ models, such as ovarian epithelium (Ohtake, Katabuchi, Matsuura, & Okamura. 1999). The differentiation of urothelium in collagen matrix is also dependent on the formation of a basement matrix specifically driven by stromal interactions, and not by soluble stromal factors (Fujiyama, Masaki, & Sugihara. 1995). The breast and ovarian models discussed above all found evidence of a complete basal lamina forming beneath the epithelium, whilst our results found only an incomplete basal lamina suggesting that Matrigel alone was sufficient to induce differentiation, a phenomenon also reported with rat prostate (Ma, Fujiyama, Masaki, & Sugihara. 1997).

Previous attempts to produce the prostatic model described here have failed to produce morphological differentiation, most likely due to the absence of stromal cultures. Early attempts using primary rat epithelia (Freeman, Bagli, Lamb, et al. 1994) and more recently human primary epithelia (Hudson, O'Hare, Watt, & Masters. 2000) both successfully grew spheroids in serum free media and in both instances spheroids of solid cells exhibiting a phenotype of hyperplastic growth were produced. Such morphologies were evident even in the presence of soluble stromal factors and the expression of androgen receptor (Hudson, O'Hare, Watt, & Masters. 2000). In contradiction of this, prostatic cell lines can undergo morphological and functional differentiation in Matrigel when plated without stroma (Webber, Bello, Kleinman, & Hoffinan. 1997), suggesting the immortalisation process can override the requirement for stromal cells to induce full differentiation as described here. The importance of mesenchyme for epithelial differentiation is fundamental and has been demonstrated by numerous animal studies (Timms, Lee, Aumüller, & Seitz. 1995)17). Mesenchyme from different origins can induce epithelia to differentiate along different pathways. For example urogenital mesenchyme can induce bladder epithelium to undergo prostatic differentiation, indicating the potential existence of a urogenital stem cell (39). More recently, a greater contribution of stroma towards the development of disease is being considered. Studies have shown that stroma from different reproductive states of the of the breast (Bemis & Schedin. 2000) or prostate tumours (Lang,SH., Stower,M. and Maitland,NJ. (2000) In vitro modelling of epithelial and stromal interactions in non-malignant and malignant prostates. British Journal of Cancer 82(4): 990-997 , Hall, J., Maitland, N.J., Stower, M., Lang, S., (2001) Primary Prostate Stromal Cells Modulate the Morphology and Migration of Primay Prostate Epithelial Cells in Type 1 Collagen Gels. Cancer Research 62: 58-62) can modulate invasion and motility of the epithelium, characteristics clearly important for cancer progression. Our model will provide a useful tool for studying how epithelial/stromal interactions contribute towards cancer progression.

The formation of spheroids in Matrigel in the presence of serum had distinct effects on the phenotypic profile of the epithelium. Spheroids grown in serum free media showed two distinct cellular compartments. The outer cells of the spheroid were basal in morphology and phenotype (cytokeratin 1,5,10,14+/cytokeratin 18-/CD44+/$\beta$1 integrin+), whilst the central cells were intermediate (cytokeratin 1,5,10,14+/cytokeratin 18+/CD44-/$\beta$1 integrin-) or luminal in phenotype (cytokeratin 1,5,10,14-/cytokeratin 18+/CD44-/$\beta$1 integrin-). The presence of PSA in all the cell populations indicates that the basal-like cells are more likely early intermediate in phenotype. These serum free spheroids are similar to those previously produced by Hudson et al (21) and also to the budding structures produced in monolayer culture by van Leenders et al (42). Phenotypically the spheroids produced in serum free conditions are more similar to in vivo acini since they contain separate cellular compartments (basal and luminal-like layers). The lack of lumen and columnar, luminal epithelium means they are morphologically dissimilar. Spheroids grown in the presence of serum, hormones and stroma produced spheroids which are morphologically very similar to in vivo acini. Phenotypically they show intermediate (cytokeratin 1,5,10,14+/cytokeratin 18+/PSA+/AR+) or luminal-like (cytokeratin 1,5,10,14-/cytokeratin 18+/PSA+/AR+) epithelial profiles but the presence of a distinct basal layer is lost. The presence of androgen receptor and a more complete morphology indicates these spheroids are more differentiated than those grown in serum free conditions. It is possible that the majority of spheroids (grown under any conditions) are derived from early basal cells and only a few are derived from stem cell populations. Those derived from early basal cells would have the capacity to differentiate but not replace a basal cell population. Those elusive spheroids derived from stem cells may therefore contain basal and luminal cells in a morphologically differentiated spheroid. It is highly likely that a proportion of the primary epithelium used in these studies were stem cell-like (or early basal cells) since they were proliferative and pluripotent (capable of producing both basal and luminal cells, stratified, columnar or cuboidal cells and also acinus-like or duct-like structures). Spheroids derived from CD44+ (basal) cells certainly gave rise to PSA+/cytokeratin 18+/CD44- (luminal) cells. This study provides further evidence for the hierarchical relationship in which basal and luminal cells are linked in a precursor progeny relationship. The heterogeneous expression of several markers of basal and luminal cells suggest that the putative stem cell population, reside within the basal layer and give rise to intermediate cells (cytokeratin 1,5,10,14+/cytokeratin 18+/PSA+) and terminally differentiated cells (cytokeratin 18+/PSA+/AR+).

Our experiments showed a variation between tissue samples. This is not unexpected given the diversity of tissue samples and the heterogeneous nature of prostatic disease. Indeed this type of analysis will bring us closer to the phenotype of prostate tumours than the analysis of a few cell lines. The age of the patient from which the tissue was obtained also plays a role in culture. In our experiments (table 1) culture of tissue from 70-90 year olds was less successful than that from younger patients (54/57 years old). This may indicate that the model requires a viable stem cell population, since stem cells will be more predominant in younger tissue. Thus our future studies will concentrate on the use of younger tissue whilst trying to analyse the contribution of age and stem cell populations to the formation of acinus-like structures.

## EXAMPLE 5

### Morphology of cell lines in monolayer

All the cell lines showed a typical epithelial morphology, which was round or cuboidal and became cobblestone-like when confluent (figure 11). Untypically, all cell lines also showed the presence of pseudopodial extensions in a percentage of the cell population, particularly when subconfluent. Shmac 2 cells were notable for having a stringy and vacuolated appearance. Shmac 3 cells were the largest in appearance. Shmac 4 cells were notable for the appearance of blebs at the cell membrane.

## EXAMPLE 6

### Growth in monolayer

Shmac 3 cells did not grow successfully beyond 3 or 4 passages after immortalisation, thus further experiments were not attempted. Shmac 2 cells were not used beyond passage 10, whilst Shmac 4,5,6 and P4E6 were not used beyond passage 15. Figure 12 indicates the growth of all other cell lines in K2 over a period of 17 days. Overall Shmac 6 and P4E6 grew very quickly and soon reached confluence. Doubling times were just over 24 hours for P4E6 and 48 hours for Shmac 6. Shmac 5 cells grew slowly to start with a doubling time of approximately 5 days but then grew more quickly to confluence. Shmac 2 and 4 grew very slowly and did not reach confluence after the 17 day culture period.

## EXAMPLE 7

### Invasion and motility of cell lines

The potential metastatic ability of the Shmac cells was investigated by measuring their motility and invasive ability, in vitro. Shmac 5 was the only cell line capable of invasion through a Matrigel coated insert. MDA-MB-231 and P4E6 cells were included as positive and negative controls respectively. In contrast, all cell lines showed high levels of motility, though this was mainly confined to ruffling of the cell membrane. Shmac 4 cells showed lots of translation as a scattered colony, whilst only Shmac 5 and 6 were capable of individual cell translation. The invasion and motility of P4E6, PNT2-C2 and PC-3 have been measured before (Lang et al, 2001) but were included for comparison.

## EXAMPLE 8

### Phenotype of cell lines in monolayer

The cellular phenotype of the cell lines was determined by immunocytochemistry. We examined a standard variety of cellular markers, as previously reported {25970}. Cytokeratins 1,5,10,14, the $\beta1$ integrin family and CD44 are all markers of basal prostatic epithelium, whilst cytokeratin 18, prostate specific antigen (PSA), prostate specific membrane antigen (PSMA) and androgen receptor are all markers of luminal or functionally differentiated prostate epithelia. In addition the cell adhesion marker, E-cadherin and mesenchymal marker vimentin were also investigated. The results for all Shmac cells are summarized in table 2 (P4E6 are included for comparison) and an example of each stain is demonstrated for the Shmac 5 cell line in figure 14.

All cell lines except Shmac 5 and P4E6 showed very strong cytokeratin 8 staining in the cytoplasm. For Shmac 5 and Shmac 2 this staining was confined to roughly 50% of the cell population and was seen most strongly expressed by cells which were uppermost in the culture. Stronger expression was also noted for Shmac 4 and 6 in cells which were sat on other cells in a monolayer culture. Conversely, most cell lines showed weak basal cytokeratin staining, except Shmac 5 and Shmac 4. Vimentin staining was moderate or strong in all cell lines. PSA expression was weak in most cell lines except P4E6 and Shmac 4 where it was moderate. PSMA was moderate to strong in all and was expressed in the cytoplasm or located to the cell membrane. No androgen receptor expression was detected in any cell line, though a minority of cells may have shown weak expression (see figure 14E).The basal markers CD44 and $\beta1$ integrin were strongly expressed by nearly all the cell lines and staining was membrane or cell to cell or cytoplasmic. Similarly E-cadherin expression was found in all cell lines and the cell to cell expression indicated the protein was functional.

## EXAMPLE 9

### Growth and morphology of Shmac cell lines and P4E6 grown in Matrigel

Previous investigations of the common prostate cell lines found that only PC-3 (not PNT2-C2, PNT1a, DU145 and LNCaP) could form spheroids in Matrigel that resemble in vivo acini (Lang et al 2001a and unpublished results). To determine what cellular factors are important to establish in vitro acini we examined the wider range of Shmac cell lines and P4E6. Experiments with primary cultures indicated that stromal co-culture could enhance differentiation of the in vitro acini therefore cells were plated with and without stromal coculture.

Shmac 4 cells did not form spheroids large enough to form lumen after 14 days in Matrigel culture. Stromal co-culture increased the number of spheroids forming from the culture of Shmac 5, 6 and P4E6 (Figure 15), but had little effect on spheroid formation from Shmac 2 cells. Figures 16a and 16b show the phase images and sections of spheroids grown from cells plated into Matrigel after 7-10 days in culture. Shmac 2 and P4E6 cells formed large spheroids in the absence of stroma, sectioning revealed them to be mutilayered. Stromal coculture reduced their size and led to the loss of lumen. Shmac 5 and 6 cells also formed smaller spheroids in the presence of stroma, though the differences were less apparent. Shmac 5 cells formed acinus-like spheroids both with and without stromal co-culture and had predominantly single layers of epithelia. Examination of several sections indicated the epithelia grown in the presence of stroma were predominantly columnar or cuboidal, whereas culture in the absence of stroma produced cuboidal or stratified cells. Examination of single epithelia within Shmac 5 spheroids grown with stroma indicated the cells showed luminal polarization of microvilli, secretory vesicles and Golgi (figure 17). Golgi were notably extensive throughout the cytoplasm. Nuclei were mainly basal in position. In addition, desmosomal-like junctions were visible between adjacent cells. The lumen showed very little cellular debris or necrotic cells. Shmac 5 cells grown in the absence of stroma could also exhibit polarization of intracellular organelles, though this was less frequently observed. Shmac 6 cells produced small spheroids which showed no evidence of lumen.

## EXAMPLE 10

### Phenotype of Shmac 5 Matrigel spheroids

Shmac 5 Matrigel spheroids were further investigated by immunocytochemical analysis, results are summarized in table 3. In particular, evidence of cellular polarization was examined (figure 18). The Shmac 5 spheroids demonstrated a phenotype very similar to primary epithelial cell spheroids co-cultured with stroma {25970}. The outer cells of the spheroid stained for basal cytokeratins, whilst the inner cells stained for luminal cytokeratins or co-localised for both. Androgen receptor expression was now apparent throughout the cytoplasm of all the cells in the spheroid, and accasionally in the nucleus. There was little localisation of PSA expression to the lumen and little basal expression of $\beta1$ integrins. However, CD44 did localize to the basal surface of the spheroid. PSMA expression was cytoplasmic or membrane specific and E-cadherin was cytoplasmic or found at cell:cell membranes indicating it was functional.

## EXAMPLE 11

### Immortalisation or extended life span of E6 Transformed Prostate Epithelial Cells?

Introduction of the E6 gene into a primary prostatic cell culture serves to extend the lifespan of the cells. For prostatic epithelium, any extension beyond passage 3-4 represents an extension of life-span. In the initial stages, the cells are genetically stable and resemble the original culture in morphology (see Figure 19). The cells are NOT however immortal at this stage, and require to pass through a *crisis* for full immortalisation to occur. After the crisis period, the cells are still epithelial in morphology, although certain chromosomal rearrangements will have occurred.

The extended lifespan cells, produced after the first retroviral infection, are genetically stable, and behave in a very similar way to the original primary cells in most of the biological assays for up to 12 population doublings. If extremely large cell numbers are not required, then the extended life span cells are preferable. To maintain these cells, a proportion of the culture should be preserved by standard cryo-preservation at every passage, particularly while the cells are proliferating.

## EXAMPLE 12

### Passage numbers

[0104]   For most of the E6 extended life span cultures, at least 25 passages are possible after introduction of the E6 gene and selection of cell clones. This is unpredictable, and may depend on the age of the patient. We have seen no relationship to the tumorigenic phenotype however. Almost inevitably, after up to 30 passages the epithelial cells enter a crisis, and a prolonged G0 phase. The essential requirement at this point is the patience to maintain the cultures for periods up to 6 weeks, feeding essentially static and apparently dead cells. Spontaneously immortalised cells emerge from this crisis infrequently, frequently resembling the original culture, but with a less epithelial phenotype (expression of vimentin - a stromal marker- is sometimes upregulated compared to the original culture)

References

1. McNeal, J.E. Normal histology of the prostate. Am. J. Surg. Path., 12: 619-633, 1988.

2. El-Alfy, M., Pelletier, G., Hermo, L.S., and Labrie, F. Unique features of the basal cells of human prostate epithelium. Microscopy Research and Technique, 51: 436-446, 2000.

3. De Marzo, A.M., Nelson, W.G., Meeker, A.K., and Coffey, D.S. Stem cell features of benign and malignant prostate epithelial cells. J. Urology, 160: 2381-2392, 1998.

4. Timms, B.G., Lee, C.W., Aumüller, G., and Seitz, J. Instructive induction of prostate growth and differentiation by a defined urogenital sinus mesenchyme. Microscopy Research and Technique, 30: 319-332, 1995.

5. Kinbara, H., Cunha, G.R., Boutin, E., Hayashi, N., and Kawamura, J. Evidence of stem cells in the adult prostatic epithelium based upon responsiveness to mesenchymal inductors. Prostate, 29: 107-116, 1996.

6. Liu, A.Y., True, L.D., LaTray, L., Nelson, P.S., Ellis, W.J., Vessella, R.L., Lange, P.H., Hood, L., and Van den Engh, G. Cell-cell interaction in prostate gene regulation and cytodifferentiation. PNAS USA, 94: 10705-10710, 1997.

7. Bayne, C.W., Donnelly, F., Chapman, K., Bollina, P., Buck, C., and Habib, F.K. A novel coculture model for benign prostatic hyperplasia expressing both isoforms of $5\alpha$-reductase. J. Clin. Endo. Met. 83: 206-213, 1998.

8. Webber, M.M., Bello, D., Kleinman, H.K., and Hoffman, M.P. Acinar differentiation by non-malignant immortalized human prostatic epithelial cells and its loss by malignant cells. Carcinogenesis, 18: 1225-1231, 1997.

9. Howlett, A.R., Hodges, G.M., and Rowlatt, C. Epithelial-stromal interactions in the adult bladder: urothelial growth, differentiation, and maturation on culture facsimiles of bladder stroma. Dev. Biology, 118: 403-415, 1986.

10. Schoop, V.M., MIrancea, N., and Fusenig, N.E. Epidermal organization and differentiation of HaCaT keratinocytes in organotypic coculture with human dermal fibroblasts. J. Invest. Dermatology, 112: 343-353, 1999.

11.Barcellos-Hoff, M.H., Aggeler, J., Ram, T.G., and Bissell, M.J. Functional differentiation and alveolar morphogenesis of primary mammary cultures on reconstituted basement membrane. Development, 105: 223-235,1989.

12. Darcy, K.M., Zangani, D., Shea-Eaton, W., Shoemaker, S.F., Lee, P.P.H., Mead, L.H., Mudipalli, A., Megan, R., and Ip, M.M. Mammary fibroblasts stimulate growth, alveolar morphogenesis, and functional differentiation of normal rat mammary epithelial cells. In Vitro Cell. Dev. Biology: Animal, 36: 578-592, 2000.

13. Boyle, P. New insights into the epidemiology and natural history of benign prostatic hyperplasia. In K. Kurth and D.W.W. Newling (Eds.), Benign Prostatic Hyperplasia, pp. 3-18. Wiley-Liss Inc, 1994.

14. Dijkman, G.A., and Debruyne, F.M.J. Epidemiology of prostate cancer. Eur. Urology, 30: 281-295, 1996.

15. Hayward, S.W., Rosen, M.A., and Cunha, G.R. Stromal-epithelial interactions in the normal and neoplastic prostate. Br. J. Urol., 79: 18-26, 1997.

16. Condon, M.S., and Bosland, M.C. The role of stromal cells in prostate cancer development and progression. In Vivo, 13: 61-65, 1999.

17. Hayward, S.W., Haughney, P.C., Lopes, E.S., Danielpour, D., and Cunha, G.R. The rat prostatic epithelial cell line NRP-152 can differentiate in vivo in response to its stromal environment. Prostate, 39: 205-212, 1999.

18. Darcy, K.M., Black, J.D., Hahm, H.A., and Ip, M.M. Mammary organoids from immature virgin rats undergo ductal and alveolar morphogenesis when grown within a reconstituted basement membrane. Exp. Cell Res., 196: 49-65,1991.

19. Ma, Y.L., Fujiyama, C., Masaki, Z., and Sugihara, H. Reconstruction of prostatic acinus-like structure from ventral and dorsolateral prostatic epithelial cells of the rat in three- dimensional collagen gel matrix culture. J. Urol., 157: 1025-1031, 1997.

20. Freeman, M.R., Bagli, D.J., Lamb, C.C., Guthrie, P.D., Uchida, T., Slavin, R.E., and Chung, L.W.K. Culture of a prostatic cell line in basement membrane gels results in an enhancement of malignant properties and constitutive alterations in gene expression. J. Cell. Physiology, 158: 325-336, 1994.

21. Hudson, D.L., O'Hare, M., Watt, F.M., and Masters, J.R.W. Proliferative heterogeneity in the human prostate: Evidence for epithelial stem cells. Lab. Invest., 80: 1243-1250, 2000.

22. Lipschutz, J.H., Foster, B.A., and Cunha, G.R. Differentiation of rat neonatal ventral prostates grown in a serum-free organ culture system. Prostate, 32: 35-42, 1997.

23. Blanchere, M., Berthaut, I., Portois, M.-C., Mestayer, C., and Mowszowicz, I. Hormonal regulation of the androgen receptor expression in human prostatic cells in culture. J. Ster. Biochem., 66: 319-326, 1998.

24. Collins, A.T., Zhiming, B., Gilmore, K., and Neal, D.E. Androgen and oestrogen responsiveness of stromal cells derived from human hyperplastic prostate: oestrogen regulation of the androgen receptor. J. Endo., 143: 269-277, 1994.

25. Paradis, V., Eschwège, P., Loric, S., Dumas, F., Ba, N., Benoit, G., Jardin, A., and Bedossa, P. De novo expression of CD44 in prostate carcinoma is correlated with systemic dissemination of prostate cancer. J. Clin. Pathology, 51: 798-802, 1998.

26. Nagle, R.B. Intermediate filament expression in prostate cancer. Can. Met. Revs., 15: 473-482, 1996.

27. Robinson, E.J., Neal, D.E., and Collins, A.T. Basal cells are progenitors of luminal cells in primary cultures of differentiating human prostatic epithelium. Prostate, 37: 149-160, 1998.

28. Knox, J.D., Cress, A.E., Clark, V., Manriquez, L., Affinito, K.-S., Dalkin, B.L., and Nagle, R.B. Differential expression of extracellular matrix molecules and the α6-integrins in the normal and neoplastic prostate. Am. J. Path., 145: 167-174, 1994.

29. Iwatsuki, H., Sasaki, K., Suda, M., and Itano, C. Vimentin intermediate filament protein as differentiation marker of optic vesicle epithelium in the chick embryo. Acta Histochemica, 101: 369-382, 1999.

30. Chang, S.S., Reuter, V.E., Heston, W.D.W., Bander, N.H., Grauer, L.S., and Gaudin, P.B. Five different anti-prostate-specific membrane antigen (PSMA) antibodies confirm PSMA expression in tumor-associated neovasculature. Cancer Res., 59: 3192-3198, 1999.

31. Anidjar, M., Villette, J.M., Devauchelle, P., Delisle, F., Cotard, J.P., Billotey, C., Cochand-Priollet, B., Copin, H., Barnoux, M., Triballeau, S., Rain, J.D., Fiet, J., Teillac, P., Berthon, P., and Cussenot, O. In vivo model mimicking natural history of dog prostate cancer using DPC-1, a new canine prostate carcinoma cell line. Prostate, 46:2-10, 2001.

32. Hayward, S.W., Del Buono, R., Deshpande, N., and Hall, P.A. A functional model of adult human prostate epithelium. The role of androgens and stroma in architectural organisation and the maintenance of differentiated secretory function. J. Cell Science, 102:361-372, 1992.

33. Sato, N., and Takahashi, H. Hepatocyte growth factor promotes growth and lumen formation of fetal lung epithelial cells in primary culture. Respirology, 3: 185-191, 1999.

34. Chen, L.-H., and Bissell, M.J. A novel regulatory mechanism for whey acidic protein gene expression. Cell Regulation, 1: 45-54, 1989.

35. Gomm, J.J., Coope, R.C., Browne, P.J., and Coombes, R.C. Separated human breast epithelial and myoepithelial cells have different growth factor requirements in vitro but can reconstitute normal breast lobuloalveolar structure. J. Cell. Physiology, 171: 11-19, 1997.

36. Niemann, C., Brinkmann, V., Spitzer, E., Hartmann, G., Sachs, M., Naundorf, H., and Birchmeier, W. Reconstitution of mammary gland development in vitro: requirement of c-met and c-erbB2 signalling for branching and alveolar morphogenesis. J. Cell Biology, 143: 533-545, 1998.

37. Ohtake, H., Katabuchi, H., Matsuura, K., and Okamura, H. A novel in vitro experimental model for ovarian endometriosis: the three-dimensional culture of human ovarian surface epithelial cells in collagen gels. Fertility and Sterility, 71: 50-55,1999.

38. Fujiyama, C., Masaki, Z., and Sugihara, H. Reconstruction of the urinary bladder mucosa in three-dimensional collagen gel culture: fibroblast-extracellular matrix interactions on the differentiation of transitional epithelial cells. J. Urology, 153: 2060-2067, 1995.

39. Cunha, G.R., Lung, B., and Reese, B. Glandular epithelial induction by embryonic mesenchyme in adult bladder epithelium of BALB/C mice. Inv. Urology, 17: 302-304, 1980.

40. Bemis, L.T., and Schedin, P. Reproductive state of rat mammary gland stroma modulates human breast cancer cell migration and invasion. Cancer Res., 60: 3414-3418,2000.

41. Lang, S.H., Stower, M., and Maitland, N.J. In vitro modelling of epithelial and stromal interactions in non-malignant and malignant prostates. Br. J. Cancer, 82: 990-997,2000.

42. van Leenders, G., Dijkman, H., Hulsbergen van de Kaa, C., Ruiter, D., and Schalken, J. Demonstration of intermediate cells during human prostate epithelial differentiation in situ and in vitro using triple-staining confocal scanning microscopy. Lab. Invest., 80: 1251-1258, 2000.

43. Lang, S.H., Clarke, N.W., George, N.J.R., Allen, T.D., and Testa, N.G. Interaction of prostate epithelial cells from benign and malignant tumor tissue with bone-marrow stroma. Prostate, 34: 203-213, 1998.

44. Chaproniere, D.M., & McKeehan, W.L. Serial culture of single adult human prostatic epithelial cells in serum-free medium containing low calcium and new growth factor from bovine brain. Cancer Res., 46: 819-824, 1986.

45. Allen, T.D and de Wynter, E.A. Visualization of haemopoietic and stromal cell types and their interactions in bone marrow culture. In: N.G. Testa and G. Molineux, (eds.), Haemopoiesis. A practical approach, pp201-218. IRL Press, Oxford, UK, 1993.

**Claims**

1.  An *in vitro* method for the formation of prostate-like acini comprising:

    i) providing a cell culture vessel comprising:

        a) prostate derived epithelial cells;
        b) a collagen-based cell support matrix to which the cells in (a) can attach and proliferate;
        c) cell culture medium supplemented with serum, a prostate stromal fraction comprising stromal cells and the hormones oestrogen and dihydrotestosterone provided at about 10ng/ml and about $10^{-7}$M respectively;

    ii) providing conditions which promote the growth and differentiation of said prostate derived cells in said vessel.

2.  A method according to Claim 1 wherein said stromal fraction is provided in a separate insert in said cell culture vessel, but in liquid contact with the other components of the supplemented cell culture medium which allows said cells contained in said stromal fraction to proliferate but prevents cell contact with the prostate derived epithelial cells contained in said vessel.

3.  A method according to Claims 1 our 2 wherein said prostate cells are human epithelial cells.

4.  A method according to any of Claims 1-3 wherein said epithelial cells are derived from prostate glands which have been maintained as explants for at least 7 days.

5.  A method according to any of Claims 1-4 wherein said prostate derived epithelial cells are non-cancerous.

6.  A method according to any of Claims 1-4 wherein said prostate derived epithelial cells are cancerous.

7.  A method according to any of Claims 1-6 wherein said epithelial cells are primary prostate epithelial cells.

8.  A method according to any of Claims 1-7 wherein said prostate derived epithelial cells are genetically engineered by recombinant techniques.

9.  A method according to Claim 8 wherein said prostate derived epithelial cells are transformed with an oncogene.

10. A method according to Claim 9 wherein said oncogene is a viral oncogene.

11. A method according to Claim 10 wherein said viral oncogene is selected from the group consisting of: Human Papilloma Virus (HPV) E6 or E7 oncogenes, SV40 T antigen.

12. A method according to any of Claims 1-11 wherein serum is provided at between 0.5%- 4% (v/v).

13. A method according to Claim 12 wherein serum is provided at between 1%-3% (v/v).

14. A method according to Claims 12 or 13 wherein serum is provided at about 2% (v/v).

15. A method according to any of Claims 1-14 wherein oestrogen is provided at 10ng/ml and dihydrotestosterone at $10^{-7}$M.

16. A cell culture composition comprising a collagen based cell support; prostate stroma comprising prostate stromal cells, oestrogen and dihydrotestosterone wherein oestrogen is provided at about 10ng/ml and dihydrotestosterone at about $10^{-7}$M.

**Patentansprüche**

1.  *In vitro*-Verfahren zur Bildung von prostata-artigen Azini, umfassend:

    i) Bereitstellen eines Zellkulturgefäßes, umfassend:

    a) von Prostata stammende Epithelzellen;
    b) eine Trägermatrix für Zellen auf Kollagenbasis, an die die Zellen von (a) adhärieren und sich vermehren können;
    c) Zellkulturmedium, das mit Serum supplementiert ist, eine Stromazellen umfassende Stromafraktion der Prostata, und die Hormone Östrogen und Dihydrotestosteron, bereitgestellt mit etwa 10 ng/ml bzw. etwa $10^{-1}$ M;

    ii) Vorsehen von Bedingungen, die das Wachstum und die Differenzierung der von der Prostata stammenden Zellen in dem Gefäß fördern.

2.  Verfahren nach Anspruch 1, wobei die Stromafraktion in einem separaten Einsatz in dem Zellkulturgefäß bereitgestellt wird, aber mit den anderen Komponenten des supplementierten Zellkulturmediums in flüssigem Kontakt steht, was es den Zellen, die in der Stromafraktion enthalten sind, ermöglicht, sich zu vermehren, aber einen Zellkontakt mit den von der Prostata stammenden Epithelzellen, die in dem Gefäß enthalten sind, verhindert.

3.  Verfahren nach den Ansprüchen 1 oder 2, wobei die Prostatazellen humane Epithelzellen sind.

4.  Verfahren nach einem der Ansprüche 1-3, wobei die Epithelzellen von Prostatadrüsen stammen, die wenigstens 7 Tage als Explantate gehalten wurden.

5.  Verfahren nach einem der Ansprüche 1-4, wobei die von der Prostata stammenden Zellen nicht kanzerogen sind.

6.  Verfahren nach einem der Ansprüche 1-4, wobei die von der Prostata stammenden Zellen kanzerogen sind.

7.  Verfahren nach einem der Ansprüche 1-6, wobei die Epithelzellen primäre Prostata-Epithelzellen sind.

8.  Verfahren nach einem der Ansprüche 1-7, wobei die von der Prostata stammenden Epithelzellen mittels rekombinanter Verfahren gentechnisch verändert sind.

9.  Verfahren nach Anspruch 8, wobei die von der Prostata stammenden Epithelzellen mit einem Onkogen transformiert sind.

10. Verfahren nach Anspruch 9, wobei das Onkogen ein virales Onkogen ist.

11. Verfahren nach Anspruch 10, wobei das virale Onkogen ausgewählt ist aus der Gruppe bestehend aus: E6- oder E7-Onkogen von Humanem Papilloma-Virus (HPV), SV40-T-Antigen.

12. Verfahren nach einem der Ansprüche 1-11, wobei das Serum in einer Konzentration zwischen 0,5%-4% (V/V) bereitgestellt wird.

13. Verfahren nach Anspruch 12, wobei das Serum in einer Konzentration zwischen 1%-3% (V/V) bereitgestellt wird.

14. Verfahren nach Anspruch 12 oder 13, wobei das Serum in einer Konzentration von etwa 2% (V/V) bereitgestellt wird.

15. Verfahren nach einem der Ansprüche 1-14, wobei Östrogen in einer Konzentration von 10 ng/ml und Dihydrotestosteron in einer Konzentration von $10^{-7}$ M bereitgestellt wird.

16. Zellkulturzusammensetzung, umfassend einen Träger für Zellen auf Kollagenbasis; Prostatastroma, umfassend Prostata-Stromazellen, Östrogen und Dihydrotestosteron, wobei Östrogen in einer Konzentration von etwa 10 ng/ml und Dihydrotestosteron in einer Konzentration von etwa $10^{-7}$ M zur Verfügung steht.

**Revendications**

1.  Méthode in vitro de formation d'acini de type prostate comprenant les étapes :

    i) Fourniture de matériel de culture cellulaire comprenant :

    a) Des cellules épithéliales dérivées de la prostate ;
    b) Une matrice support cellulaire à base de collagène à laquelle des cellules selon (a) peuvent être attachées et proliférer ;
    c) Un milieu de culture cellulaire enrichi en sérum, une fraction du stroma de la prostate comprenant des cellules de stroma et les hormones oestrogène et dihydrotestostérone à des ratios respectifs d'environ 10 ng/ml et $10^{-7}$M;

    ii) fournir des conditions qui favorisent la croissance et la différentiation desdites cellules de la prostate dans ledit matériel.

2.  Méthode selon la revendication 1, **caractérisée en ce que** ladite fraction de stroma est fournie dans un insert séparé dans ledit matériel de culture cellulaire, mais en contact liquide avec les autres composants du milieu de culture cellulaire enrichi, ce qui permet auxdites cellules contenues dans ladite fraction de stroma de proliférer, mais empêche un contact cellulaire avec les cellules épithéliales dérivées de la prostate contenues dans ledit matériel.

3.  Méthode selon les revendications 1 ou 2, **caractérisée en ce que** lesdites cellules de prostate sont des cellules épithéliales humaines.

4.  Méthode selon l'une quelconque des revendications 1 à 3, **caractérisée en ce que** lesdites cellules épithéliales sont dérivées de glandes prostatiques qui ont été maintenues comme explants pendant au moins 7 jours.

5.  Méthode selon l'une quelconque des revendications 1 à 4, **caractérisée en ce que** lesdites cellules épithéliales dérivées de la prostate ne sont pas cancéreuses.

6.  Méthode selon l'une quelconque des revendications 1 à 4, **caractérisée en ce que** lesdites cellules épithéliales dérivées de la prostate sont cancéreuses.

7.  Méthode selon l'une quelconque des revendications 1 à 6, **caractérisée en ce que** lesdites cellules épithéliales sont des cellules épithéliales primaires prostatiques.

8.  Méthode selon l'une quelconque des revendications 1 à 7, **caractérisée en ce que** lesdites cellules épithéliales dérivées de la prostate sont modifiées génétiquement par des techniques recombinantes.

9.  Méthode selon la revendication 8, **caractérisée en ce que** lesdites cellules épithéliales dérivées de la prostate sont transformées avec un oncogène.

10. Méthode selon la revendication 9, **caractérisée en ce que** ledit oncogène est un oncogène viral.

11. Méthode selon la revendication 10, **caractérisée en ce que** ledit oncogène viral est choisi parmi le groupe des oncogènes E6 et E7 du virus humain papillome (HPV), de l'antigène SV40T.

12. Méthode selon l'une quelconque des revendications 1 à 11, **caractérisée en ce que** le sérum est fourni à entre 0,5% et 4% (v/v).

13. Méthode selon l'une quelconque des revendications 1 à 12, **caractérisée en ce que** le sérum est fourni à entre 1% et 3% (v/v).

14. Méthode selon l'une des revendications 12 ou 13, **caractérisée en ce que** le sérum est fourni à environ 2% (v/v).

15. Méthode selon l'une quelconque des revendications 1 à 14, **caractérisée en ce que** l'oestrogène est fourni à 10 ng/ml et la dihydrotestotérone à $10^{-7}$M.

**16.** Composition de culture cellulaire comprenant un support cellulaire à base de collagène, du stroma de prostate comprenant des cellules de stroma prostatiques, de l'oestrogène et de la dihydrotestostérone, **caractérisée en ce que** l'oestrogène est fourni à environ 10 ng/ml et la dihydrotestotérone à environ $10^{-7}$ M.

Table 1: Summary of epithelium/stromal co-cultures in Matrigel*

| Epithelia: sample | Age | diagnosis | Preparation | Stroma[†]: sample | age | Morphology in Matrigel: Spheroid | Epithelia |
|---|---|---|---|---|---|---|---|
| B | 86 | BPH | Ex | i | 80 | Ac, B, D | Cub, S |
| C | 73 | BPH | Ex | ii | 57 | CAc | Col (p), Cub |
| D | 76 | BPH | Ex | iii | 75 | Ac | nd |
| F | 78 | BPH | cd44 | iv | 74 | Irr, B | nd |
| G | 76 | BPH | cd44 | iv | " | Ac | Cub, S |
| G | " | " | Ex,cd44 | iv | " | Ac | Cub, S, Col |
| I | 75 | BPH | Ex,cd44 | v | 89 | Ac | nd |
| I | " | " | " | STO | - | Ac | nd |
| J | 54 | Normal | Ex,cd44 | vi | 72 | CAc | Col (p), Cub |
| J | " | " | " | STO | - | CAc | Col (p), Cub |

*Co-cultures were grown in K2 supplemented with $20^{-7}$M DHT and 10 ng/ml Oestrogen. [†]All stromal cultures were BPH derived. Abbreviations used were: Ex, 7 day explant culture; cd44, CD44 positive epithelial cell isolation; Irr, irregular shaped spheroids of solid cells; Ac, acinus-like spheroid containing a lumen and one/two epithelial layers; cAc, compact acinus-like spheroid; D, ducts; B, budding; Cub, cuboidal; Col, columnar; (p), polarised; S, stratified.

Table 2. Immunofluorescent phenotype of primary prostate epithelial spheroids grown in Matrigel

| Marker | KSFM | K2 | K2+S+DO |
|---|---|---|---|
| Cytokeratin 18 | ++[c] | ++ | ++ |
| Cytokeratin 1,5,10,14 | ++[o] | ++ | ++ |
| Vimentin | + | + | + |
| PSA | ++ | +++ | +++[l] |
| PSMA | +++[o] | +++ | +++ |
| androgen receptor | - | - | + |
| E-Cadherin | ++ | ++ | ++ |
| Desmoglein | ++ | ++ | ++ |
| CD44 | +++[o] | +++ | +++ |
| β1 integrin | ++[o] | +++ | +++[b] |

Abbreviations used were: +, weak staining; ++ moderate staining; +++ strong staining; l, luminal; b, basal; o, outer cells; c, central cells

## Table 3. Antibodies and Procedures for Fluorescent Immunostaining

| Antigen | Clone/sera | Supplier | Dilution | Secondary procedure |
|---|---|---|---|---|
| Cytokeratin 18 (FITC conjugate) | CY90 | Sigma | 1:20 | - |
| Cytokeratin 1, 5, 10, 14 | 34βE12 | Dako | 1:50 | RB, SF |
| Vimentin | VIM 13.2 | Sigma | 1:200 | RF |
| PSA | rabbit | Dako | 1:20 | SB, SF |
| PSMA | PSM-P12 | JM Villete | 1:100 | RF |
| androgen receptor | NCL-AR-318 | Novocastra | 1:50 | RB, SF |
| Desmoglein | CBL 174 | Cymbus Biotechnology | 1:10 | RB, SF |
| E-Cadherin | HECD-1 | R&D | 10 μg/ml | RB, SF |
| β1 integrin | sc-9970 | Santa Cruz | 2 μg/ml | RF |
| CD44 | F10 44-2 | Novocastra | 1:10 | RB, SF |

Abbreviations are as follows: RF = 1:30 dilution of FITC conjugated rabbit anti mouse; RB = 1:300 dilution of Biotinylated rabbit anti- mouse; SF = 1:50 dilution of FITC conjugated streptavidin; SB = 1:300 dilution of Biotinylated swine anti-rabbit.

Table 4: Motility of the Shmac cell lines

| Cell Line | Ruffling | Pseudopods | Translation | Motility Index |
|---|---|---|---|---|
| Shmac 2 | 2 | 1 | 1 | 4 |
| Shmac 4 | 3 | 1 | 3 | 7 |
| Shmac 5 | 3 | 1 | 1+1 | 6 |
| Shmac 6 | 3 | 1 | 1+1 | 6 |
| | | | | |
| P4E6 | 2 | 3 | 3+2 | 10 |
| PC-3 | 2 | 1 | 2 | 5 |
| PNT2-C2 | 1 | 0 | 0 | 1 |

Motility was scored as follows: Ruffling, 1,2 or 3; Pseudopodial movement, 0,1,2,3,or 4; translative movement as a colony, 1,2 or 3 and individual cell translation +1 or +2. For details see Lang et al 2000.

Table 5: Phenotype of E6 transformed cell lines in monolayer

| | Shmac 2 | Shmac 4 | Shmac 5 | Shmac 6 | P4E6 |
|---|---|---|---|---|---|
| Cytokeratin 18 | +++ 50%*,u,c | +++ c | ++ 50%,u,c | +++ c | ++ c |
| Cytokeratin 1,5,10,14 | + c | ++ c | ++ c | + c | + c |
| Vimentin | ++ c | ++ c | ++ c | +++ c | +++ c |
| PSA | + c | ++ c | + c | + c | ++ c,m |
| PSMA | +++ c,m | +++ c | ++ c | ++ c,m | ++ c |
| AR | - | - | - | - | - |
| β1 integrin | +++ c,m,c:c | +++ c,m,c:c | ++ c,m,c:c | +++ c,m,c:c | +++ m,c:c |
| CD44 | +++ c,m,c:c | +++ c,m,c:c | +++ c,m,c:c | +++ c,m,c:c | ++ m |
| E-cadherin | + c,c:c | ++ c,c:c | ++ c,c:c | + c,c:c | + c |

*100% of the cell population show positive staining unless noted

abbreviations used were: u=upper cells; c = cytoplasmic; m = membrane; c:c = cell to cell interface .

Intensity of staining was: +++ high; ++ moderate; + weak.

Table 6: Phenotype of Shmac 5 in Matrigel

|  | Shmac 5 |
|---|---|
| Cytokeratin 18 | ++ i,c |
| Cytokeratin 1,5,10,14 | ++ o,c |
| Vimentin    · | + c |
| PSA | ++ c,m |
| PSMA | ++ c,m |
| AR | ++ c |
| β1 integrin | ++ c,c:c |
| CD44 | ++ c,b |
| E-cadherin | ++ c,c:c |

abbreviations used were: i=inner cells; c = cytoplasmic; o=outer cells; m = membrane; c:c = cell to cell interface;

b=basal.

Intensity of staining was: +++ high; ++ moderate; + weak.

Fig 1.

Fig. 2

Fig 3

Fig. 4

Budding          Ducts

Phase contrast

Sections

FiG. 5

FIG. 6

KSFM        K2        K2 + D + O + S

Figure 7

Fig. 8

|  | K2 | K2+D+O+S |
| PSA | | |
| β1 integrin | | |

vimentin  PSMA  AR  E-cadherin  desmoglein  CD44

Figure 9

FiG 10

— Culture insert

— Culture well

— Culture media

— Stromal layer

— Epithelial cell
— Matrigel
— Spheroid

FiG. 11

Figure 15

Figure 13

FiG. 14

Figure 15

# Fig. 16 a

FIG. 16b

stromal
co-culture    control

Shmac 2

P4E6

Shmac 5

Shmac 6

FIG. 17

Fig. 18

FIG. 19

Fig. 20

Fig. 21

A           B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5874305 A
- US 5917124 A
- US 5907078 A
- US 5631236 A
- US 5601818 A

### Non-patent literature cited in the description

- **VAHERI ; PAGANO.** *Science,* vol. 175, 434
- **GRAHAM et al.** *Virology,* 1973, vol. 52, 456
- **FELGNER.** *Proc.Natl.Acad.Sci USA,* 1987, vol. 84, 7413
- **CAPECCHI.** *Cell,* 1980, vol. 22, 479
- **BILDIRICI et al.** *Nature,* vol. 405, 769
- **LANG,SH. ; STOWER,M. ; MAITLAND,NJ.** In vitro modelling of epithelial and stromal interactions in non-malignant and malignant prostates. *British Journal of Cancer,* 2000, vol. 82 (4), 990-997
- **HALL, J. ; MAITLAND, N.J. ; STOWER, M. ; LANG, S.** Primary Prostate Stromal Cells Modulate the Morphology and Migration of Primay Prostate Epithelial Cells in Type 1 Collagen Gels. *Cancer Research,* 2001, vol. 62, 58-62
- **MCNEAL, J.E.** Normal histology of the prostate. *Am. J. Surg. Path.,* 1988, vol. 12, 619-633
- **EL-ALFY, M. ; PELLETIER, G. ; HERMO, L.S. ; LABRIE, F.** Unique features of the basal cells of human prostate epithelium. *Microscopy Research and Technique,* 2000, vol. 51, 436-446
- **DE MARZO, A.M. ; NELSON, W.G. ; MEEKER, A.K. ; COFFEY, D.S.** Stem cell features of benign and malignant prostate epithelial cells. *J. Urology,* 1998, vol. 160, 2381-2392
- **TIMMS, B.G. ; LEE, C.W. ; AUMÜLLER, G. ; SEITZ, J.** Instructive induction of prostate growth and differentiation by a defined urogenital sinus mesenchyme. *Microscopy Research and Technique,* 1995, vol. 30, 319-332
- **KINBARA, H. ; CUNHA, G.R. ; BOUTIN, E. ; HAYASHI, N. ; KAWAMURA, J.** Evidence of stem cells in the adult prostatic epithelium based upon responsiveness to mesenchymal inductors. *Prostate,* 1996, vol. 29, 107-116
- **LIU, A.Y. ; TRUE, L.D. ; LATRAY, L. ; NELSON, P.S. ; ELLIS, W.J. ; VESSELLA, R.L. ; LANGE, P.H. ; HOOD, L. ; VAN DEN ENGH, G.** Cell-cell interaction in prostate gene regulation and cytodifferentiation. *PNAS USA,* 1997, vol. 94, 10705-10710
- **BAYNE, C.W. ; DONNELLY, F. ; CHAPMAN, K. ; BOLLINA, P. ; BUCK, C. ; HABIB, F.K.** A novel co-culture model for benign prostatic hyperplasia expressing both isoforms of 5α-reductase. *J. Clin. Endo. Met.,* 1998, vol. 83, 206-213
- **WEBBER, M.M. ; BELLO, D. ; KLEINMAN, H.K. ; HOFFMAN, M.P.** Acinar differentiation by non-malignant immortalized human prostatic epithelial cells and its loss by malignant cells. *Carcinogenesis,* 1997, vol. 18, 1225-1231
- **HOWLETT, A.R. ; HODGES, G.M. ; ROWLATT, C.** Epithelial-stromal interactions in the adult bladder: urothelial growth, differentiation, and maturation on culture facsimiles of bladder stroma. *Dev. Biology,* 1986, vol. 118, 403-415
- **SCHOOP, V.M. ; MIRANCEA, N. ; FUSENIG, N.E.** Epidermal organization and differentiation of HaCaT keratinocytes in organotypic coculture with human dermal fibroblasts. *J. Invest. Dermatology,* 1999, vol. 112, 343-353
- **BARCELLOS-HOFF, M.H. ; AGGELER, J. ; RAM, T.G. ; BISSELL, M.J.** Functional differentiation and alveolar morphogenesis of primary mammary cultures on reconstituted basement membrane. *Development,* 1989, vol. 105, 223-235
- **DARCY, K.M. ; ZANGANI, D. ; SHEA-EATON, W. ; SHOEMAKER, S.F. ; LEE, P.P.H. ; MEAD, L.H. ; MUDIPALLI, A. ; MEGAN, R. ; IP, M.M.** Mammary fibroblasts stimulate growth, alveolar morphogenesis, and functional differentiation of normal rat mammary epithelial cells. *In Vitro Cell. Dev. Biology: Animal,* 2000, vol. 36, 578-592
- New insights into the epidemiology and natural history of benign prostatic hyperplasia. **BOYLE, P.** Benign Prostatic Hyperplasia. Wiley-Liss Inc, 1994, 3-18
- **DIJKMAN, G.A. ; DEBRUYNE, F.M.J.** Epidemiology of prostate cancer. *Eur. Urology,* 1996, vol. 30, 281-295

- **HAYWARD, S.W. ; ROSEN, M.A. ; CUNHA, G.R.** Stromal-epithelial interactions in the normal and neoplastic prostate. *Br. J. Urol.,* 1997, vol. 79, 18-26
- **CONDON, M.S. ; BOSLAND, M.C.** The role of stromal cells in prostate cancer development and progression. *In Vivo,* 1999, vol. 13, 61-65
- **HAYWARD, S.W. ; HAUGHNEY, P.C. ; LOPES, E.S. ; DANIELPOUR, D. ; CUNHA, G.R.** The rat prostatic epithelial cell line NRP-152 can differentiate in vivo in response to its stromal environment. *Prostate,* 1999, vol. 39, 205-212
- **DARCY, K.M. ; BLACK, J.D. ; HAHM, H.A. ; IP, M.M.** Mammary organoids from immature virgin rats undergo ductal and alveolar morphogenesis when grown within a reconstituted basement membrane. *Exp. Cell Res.,* 1991, vol. 196, 49-65
- **MA, Y.L. ; FUJIYAMA, C. ; MASAKI, Z. ; SUGI-HARA, H.** Reconstruction of prostatic acinus-like structure from ventral and dorsolateral prostatic epithelial cells of the rat in three- dimensional collagen gel matrix culture. *J. Urol.,* 1997, vol. 157, 1025-1031
- **FREEMAN, M.R. ; BAGLI, D.J. ; LAMB, C.C. ; GUTHRIE, P.D. ; UCHIDA, T. ; SLAVIN, R.E. ; CHUNG, L.W.K.** Culture of a prostatic cell line in basement membrane gels results in an enhancement of malignant properties and constitutive alterations in gene expression. *J. Cell. Physiology,* 1994, vol. 158, 325-336
- **HUDSON, D.L. ; O'HARE, M. ; WATT, F.M. ; MASTERS, J.R.W.** Proliferative heterogeneity in the human prostate: Evidence for epithelial stem cells. *Lab. Invest.,* 2000, vol. 80, 1243-1250
- **LIPSCHUTZ, J.H. ; FOSTER, B.A. ; CUNHA, G.R.** Differentiation of rat neonatal ventral prostates grown in a serum-free organ culture system. *Prostate,* 1997, vol. 32, 35-42
- **BLANCHERE, M. ; BERTHAUT, I. ; PORTOIS, M.-C. ; MESTAYER, C. ; MOWSZOWICZ, I.** Hormonal regulation of the androgen receptor expression in human prostatic cells in culture. *J. Ster. Biochem.,* 1998, vol. 66, 319-326
- **COLLINS, A.T. ; ZHIMING, B. ; GILMORE, K. ; NEAL, D.E.** Androgen and oestrogen responsiveness of stromal cells derived from human hyperplastic prostate: oestrogen regulation of the androgen receptor. *J. Endo.,* 1994, vol. 143, 269-277
- **PARADIS, V. ; ESCHWÈGE, P. ; LORIC, S. ; DUMAS, F. ; BA, N. ; BENOIT, G. ; JARDIN, A. ; BEDOSSA, P.** De novo expression of CD44 in prostate carcinoma is correlated with systemic dissemination of prostate cancer. *J. Clin. Pathology,* 1998, vol. 51, 798-802
- **NAGLE, R.B.** Intermediate filament expression in prostate cancer. *Can. Met. Revs.,* 1996, vol. 15, 473-482
- **ROBINSON, E.J. ; NEAL, D.E. ; COLLINS, A.T.** Basal cells are progenitors of luminal cells in primary cultures of differentiating human prostatic epithelium. *Prostate,* 1998, vol. 37, 149-160
- **KNOX, J.D. ; CRESS, A.E. ; CLARK, V. ; MANRIQUEZ, L. ; AFFINITO, K.-S. ; DALKIN, B.L. ; NAGLE, R.B.** Differential expression of extracellular matrix molecules and the $\alpha$6-integrins in the normal and neoplastic prostate. *Am. J. Path.,* 1994, vol. 145, 167-174
- **IWATSUKI, H. ; SASAKI, K. ; SUDA, M. ; ITANO, C.** Vimentin intermediate filament protein as differentiation marker of optic vesicle epithelium in the chick embryo. *Acta Histochemica,* 1999, vol. 101, 369-382
- **CHANG, S.S. ; REUTER, V.E. ; HESTON, W.D.W. ; BANDER, N.H. ; GRAUER, L.S. ; GAUDIN, P.B.** Five different anti-prostate-specific membrane antigen (PSMA) antibodies confirm PSMA expression in tumor-associated neovasculature. *Cancer Res.,* 1999, vol. 59, 3192-3198
- **ANIDJAR, M. ; VILLETTE, J.M. ; DEVAUCHELLE, P. ; DELISLE, F. ; COTARD, J.P. ; BILLOTEY, C. ; COCHAND-PRIOLLET, B. ; COPIN, H. ; BARNOUX, M. ; TRIBALLEAU, S.** In vivo model mimicking natural history of dog prostate cancer using DPC-1, a new canine prostate carcinoma cell line. *Prostate,* 2001, vol. 46, 2-10
- **HAYWARD, S.W. ; DEL BUONO, R. ; DESHPANDE, N. ; HALL, P.A.** A functional model of adult human prostate epithelium. The role of androgens and stroma in architectural organisation and the maintenance of differentiated secretory function. *J. Cell Science,* 1992, vol. 102, 361-372
- **SATO, N. ; TAKAHASHI, H.** Hepatocyte growth factor promotes growth and lumen formation of fetal lung epithelial cells in primary culture. *Respirology,* 1999, vol. 3, 185-191
- **CHEN, L.-H. ; BISSELL, M.J.** A novel regulatory mechanism for whey acidic protein gene expression. *Cell Regulation,* 1989, vol. 1, 45-54
- **GOMM, J.J. ; COOPE, R.C. ; BROWNE, P.J. ; COOMBES, R.C.** Separated human breast epithelial and myoepithelial cells have different growth factor requirements in vitro but can reconstitute normal breast lobuloalveolar structure. *J. Cell. Physiology,* 1997, vol. 171, 11-19
- **NIEMANN, C. ; BRINKMANN, V. ; SPITZER, E. ; HARTMANN, G. ; SACHS, M. ; NAUNDORF, H. ; BIRCHMEIER, W.** Reconstitution of mammary gland development in vitro: requirement of c-met and c-erbB2 signalling for branching and alveolar morphogenesis. *J. Cell Biology,* 1998, vol. 143, 533-545

- **OHTAKE, H. ; KATABUCHI, H. ; MATSUURA, K. ; OKAMURA, H.** A novel in vitro experimental model for ovarian endometriosis: the three-dimensional culture of human ovarian surface epithelial cells in collagen gels. *Fertility and Sterility,* 1999, vol. 71, 50-55
- **FUJIYAMA, C. ; MASAKI, Z. ; SUGIHARA, H.** Reconstruction of the urinary bladder mucosa in three-dimensional collagen gel culture: fibroblast-extracellular matrix interactions on the differentiation of transitional epithelial cells. *J. Urology,* 1995, vol. 153, 2060-2067
- **CUNHA, G.R. ; LUNG, B. ; REESE, B.** Glandular epithelial induction by embryonic mesenchyme in adult bladder epithelium of BALB/C mice. *Inv. Urology,* 1980, vol. 17, 302-304
- **BEMIS, L.T. ; SCHEDIN, P.** Reproductive state of rat mammary gland stroma modulates human breast cancer cell migration and invasion. *Cancer Res.,* 2000, vol. 60, 3414-3418
- **LANG, S.H. ; STOWER, M. ; MAITLAND, N.J.** In vitro modelling of epithelial and stromal interactions in non-malignant and malignant prostates. *Br. J. Cancer,* 2000, vol. 82, 990-997
- **VAN LEENDERS, G. ; DIJKMAN, H. ; HULSBERGEN VAN DE KAA, C. ; RUITER, D. ; SCHALKEN, J.** Demonstration of intermediate cells during human prostate epithelial differentiation in situ and in vitro using triple-staining confocal scanning microscopy. *Lab. Invest.,* 2000, vol. 80, 1251-1258
- **LANG, S.H. ; CLARKE, N.W. ; GEORGE, N.J.R. ; ALLEN, T.D. ; TESTA, N.G.** Interaction of prostate epithelial cells from benign and malignant tumor tissue with bone-marrow stroma. *Prostate,* 1998, vol. 34, 203-213
- **CHAPRONIERE, D.M. ; MCKEEHAN, W.L.** Serial culture of single adult human prostatic epithelial cells in serum-free medium containing low calcium and new growth factor from bovine brain. *Cancer Res.,* 1986, vol. 46, 819-824
- Visualization of haemopoietic and stromal cell types and their interactions in bone marrow culture. **ALLEN, T.D ; DE WYNTER, E.A.** Haemopoiesis. A practical approach. IRL Press, 1993, 201-218